(19) Europäisches Patentamt
European Patent Office
Office européen des brevets

(11) EP 2 512 490 B1

(12) **EUROPEAN PATENT SPECIFICATION**

(45) Date of publication and mention of the grant of the patent:
**27.01.2016 Bulletin 2016/04**

(21) Application number: **10803973.6**

(22) Date of filing: **29.07.2010**

(51) Int Cl.:
*A61K 31/7076 (2006.01)* *A61K 47/06 (2006.01)*
*A61K 9/20 (2006.01)* *A61K 9/28 (2006.01)*
*A61K 47/12 (2006.01)* *A61K 47/14 (2006.01)*
*A61K 47/18 (2006.01)* *A61K 47/40 (2006.01)*

(86) International application number:
**PCT/IB2010/001877**

(87) International publication number:
**WO 2011/012989 (03.02.2011 Gazette 2011/05)**

(54) **S-ADENOSYLMETHIONINE FORMULATIONS WITH ENHANCED BIOAVAILABILITY**

S-ADENOSYLMETHIONINFORMULIERUNGEN MIT ERHÖHTER BIOVERFÜGBARKEIT

PRÉPARATIONS DE S-ADENOSYLMETHIONINE PRÉSENTANT UNE BIODISPONIBILITÉ AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **28.07.2009 US 229194 P**

(43) Date of publication of application:
**24.10.2012 Bulletin 2012/43**

(73) Proprietor: **MSI Methylation Sciences Inc.**
**British Columbia V5G 4X4 (CA)**

(72) Inventors:
- **MACDONALD, I., David**
**Surrey**
**British Columbia V4A 9W5 (CA)**
- **HARRISON, Nancy**
**North Vancouver**
**British ColumbiaV7R 3B6 (CA)**
- **TAKACS-COX, Aniko**
**North Vancouver**
**British Columbia V7G 1J3 (CA)**
- **PURAC, Admir**
**Burnaby**
**British Columbia V5J 3Z4 (CA)**
- **BLAZEK-WELSH, Almira**
**Surrey**
**British Columbia V3S 9B3 (CA)**

(74) Representative: **Gowshall, Jonathan Vallance et al**
**Forresters**
**Skygarden**
**Erika-Mann-Strasse 11**
**80636 München (DE)**

(56) References cited:
**EP-A1- 2 149 369** **EP-A1- 2 193 787**
**WO-A1-02/092112** **WO-A1-2008/095142**
**WO-A1-2010/027014** **US-A- 4 956 173**
**US-A- 6 093 703** **US-A1- 2002 164 369**
**US-A1- 2006 013 905** **US-A1- 2009 088 404**
**US-A1- 2009 110 729** **US-A1- 2009 197 824**

- **DELI ET AL: "Potential use of tight junction modulators to reversibly open membranous barriers and improve drug delivery", BIOCHIMICA ET BIOPHYSICA ACTA. BIOMEMBRANES, AMSTERDAM, NL, vol. 1788, no. 4, 1 April 2009 (2009-04-01), pages 892-910, XP026081423, ISSN: 0005-2736, DOI: 10.1016/J.BBAMEM.2008.09.016 [retrieved on 2008-10-17]**
- **HOCHMAN J ET AL: "Mechanisms of absorption enhancement and tight junction regulation", JOURNAL OF CONTROLLED RELEASE, ELSEVIER, AMSTERDAM, NL, vol. 29, no. 3, 1 March 1994 (1994-03-01), pages 253-267, XP025557175, ISSN: 0168-3659, DOI: 10.1016/0168-3659(94)90072-8 [retrieved on 1994-03-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DUIZER ERWIN ET AL: "Absorption enhancement, structural changes in tight junctions and cytotoxicity caused by palmitoyl carnitine in Caco-2 and IEC-18 cells", JOURNAL OF PHARMACOLOGY AND EXPERIMENTAL THERAPEUTICS, vol. 287, no. 1, October 1998 (1998-10), pages 395-402, XP002687013, ISSN: 0022-3565
- STRAMENTINOLI G ET AL.: "Intestinal absorption of S-adenosyl-L-methionine", THE JOURNAL OF PHARMACOLOGY AND EXPERIMENTAL THERAPEUTICS, vol. 209, no. 3, 1979, pages 323-326,
- BOTTIGLIERI T ET AL.: "Transmethylation in depression", THE ALABAMA JOURNAL OF MEDICAL SCIENCES, vol. 25, no. 3, 1988, pages 296-301,
- GIULIDORI P ET AL.: "Pharmacokinetics of S-adenosyl-L-methionine in healthy volunteers", EUR. J. CLIN. PHARMACOL., vol. 27, 1984, pages 119-121,

## Description

### TECHNICAL FIELD

The invention relates to compositions and methods for improved bioavailability of S-adenosyl-L-methionine ("SAM-e" or "SAMe"). More particularly, the invention concerns formulations that modulate absorption of exogenous SAMe in the gastrointestinal tract and that provide, through oral administration or like method, a SAMe plasma concentration from which sufficient physiological effects can be expected. The invention is directed to methods of treating a disease or disorder in a subject and/or improving the nutritional status of a subject by administering formulations enabling improved gastrointestinal absorption of SAMe, wherein increased gastrointestinal absorption is achieved using one or more absorption-enhancing technologies.

### BACKGROUND OF THE INVENTION

S-adenosyl-L-methionine ("SAM-e" or "SAMe") is a naturally occurring compound that is present in tissues throughout the body. At the molecular level, SAMe is involved in various metabolic pathways, including transmethylation, transsulfuration and aminopropylation (e.g. in the production of polyamines, such as spermidine and spermine, from putrescine).

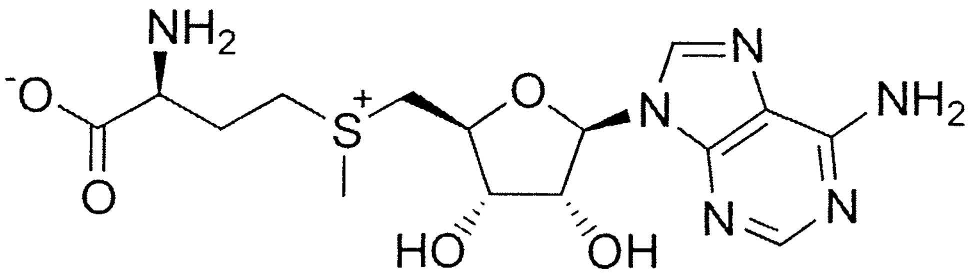

S-adenosyl-L-methionine (SAMe)

In the body, SAMe is synthesized from an amino acid, methionine, and a triphosphate nucleotide, ATP. SAMe has been tested in numerous clinical trials for the treatment of various ailments, including arthritis, liver disease and depression.

SAMe supplementation was initially considered impractical, due to the instability of the SAMe ion during manufacturing, shipping and storage. Eventually stable salts of SAMe were developed (such as SAMe tosylate disulfate, the butanedisulfonate salt of SAMe, the dipara-toluene sulfonate disulfate salt of SAMe, the tri-para-toluene sulfonic acid salt of SAMe and the like). These salts can be formulated using standard, known technologies used for non-parenteral administration including, but not limited to, tablets, capsules and pellets. Formulations such as these may also comprise a coating which can serve multiple purposes such as improving taste and ease of swallowing as well as reducing stomach irritation. Stable salts of SAMe are described in, for example, United States Patent Numbers 3,954,726 and 4,057,686. Conventional SAMe API is supplied as a molecular entity comprising an ion along with several counter-ions. For example, SAMe ion plus a tosylate and 2 sulfonic acid counter-ions make up commercially available adenosylmethionine disulfate-*p*-toluenesulfonate (*i.e.* SAMe tosylate disulfate). When referring to SAMe dosing, it is currently accepted in the art that the numerical dose (usually in milligrams) refers to the amount of SAMe ion which is administered. For example, reference to a "400 mg SAMe tablet" using SAMe tosylate disulfate would include the 400 mg of SAMe ion, another 370 mg of the counter-ions, and 200-300 mg of additional excipient to make up a final tablet weight of 1.0-1.1 grams. Thus, for example, a 1600 mg oral dose of SAMe which is generally reported in the art would typically be a dose of four such 1.0-1.1 gram tablets taken at one time. Alternatively, the same 1600 mg dose of SAMe ion may also be accomplished by administration of other combinations of multiple tablets such as, sixteen 100 mg or eight 200 mg tablets of SAMe ion taken at a given time. Conventional oral dosage forms of SAMe are most commonly produced with about 400 mg of SAME ion; above that, the larger dosage form becomes difficult for swallowing considering that even at 400 mg of SAMe ion the tablets are quite large at 1.0-1.1 grams.

The prevailing conventional wisdom in the art is of the view that gastric juices in the stomach will alter the structure and/or function of SAMe thereby reducing its absorption and therefore a pH-specific coating which bypasses any SAMe release in the stomach is deemed necessary for oral administration. These "enteric" coatings are well known and routinely used by those of skill in the art. Enteric coatings provide a barrier which protects the encapsulated agent from the extremely low pH environment of the stomach. Although 'pH-sensitive', these coatings are designed solely to

protect the encapsulated agent from the stomach. They generally begin to dissolve at a pH above about 5.5 (designed to match the pH of the environment immediately following the stomach) to allow release of the underlying dosage form. Various attempts to improve the stability and delivery of enteric coated SAMe have been reported. Rao *et al.,* describe the use of an enteric coated, lipophilic soft gelatin capsule which begins dissolving at pH 5.5 (U.S. Patent 6,759,395). They recommend the use of a lipophilic material to insulate SAMe salts as a means of protecting the encapsulated drug. Furthermore, they utilize a standard enteric coating in order to bypass any SAMe release in the stomach. The use of an enteric coating is not surprising; in view of prior art reports that SAMe cannot be absorbed in the stomach as it will be degraded first by gastric juices.

In addition to the reported claims in the art that SAMe is inactivated within the stomach, there is also a widely accepted belief surrounding the absorption mechanism and metabolism of this compound. Based on past clinical experimentation, SAMe is cited as being highly soluble and highly permeable yet exhibits low bioavailability. Studies using radiolabelled SAMe indicated that SAMe is readily absorbed in the GI tract; however; plasma analysis showed low bioavailability (Stramentinoli, G., (1987) The American Journal of Medicine 83(S 5A): 35-42). Therefore, those skilled in these arts assumed that SAMe's low bioavailability is caused by other factors, such as "first pass metabolism" in the liver. Over the past 20 years, numerous groups have attempted to understand SAMe bioavailability by looking at the pharmacokinetics, drug elimination and renal excretion profiles of various SAMe formulations but not the absorption mechanisms. It is routinely reported by those most knowledgeable in these arts that SAMe bioavailability when orally administered is limited to <5% because of "significant liver metabolism" prior to entering the blood (Bottiglieri et al., (1988) Alabama Journal of Medical Sciences 25(3): 296-301; Bottiglieri et al., (1997) Exp. Opin. Invest. Drugs 6(4): 417-426; Kaye et al., (1990) Drugs 40: 124-128). Additional drug elimination and renal excretion studies report that body accumulation of intact SAMe is unlikely as a cause of reduced bioavailability and instead also suggest that "active presystemic metabolism" is the cause (Giulidori and Cortellaro (1984) European Journal of Clinical Pharmacology 27: 119-121; Stramentinoli, G., (1986) Biological Methylation and Drug Design. R. T. Borchardt. New Jersey, Humana Press: 315-326). Another belief is that metabolism of SAMe occurs rapidly via transmethylation (and to a lesser extent, transsulfuration and aminoprophylation) pathways after non-parenteral administration. More specifically, the skilled practitioners of these arts proposed that the methyl group of SAMe is removed and incorporated into stable pools with low turnover rates, such as proteins and phospholipids (Bottiglieri (1997) *supra;* Stramentinoli (1987) *supra*), and therefore results in the very limited bioavailability of SAMe itself.

Active liver metabolism occurs with many drugs and typically causes a lower cap on their bioavailability as seen with SAMe. Also, there is a vast amount of clinical data reported in the art which supports ready absorption of SAMe. It has thus been the general dogma in the art that low SAMe bioavailability is due primarily to extensive first pass metabolism in the liver

A recent report looking at SAMe uptake into cells in culture finds that SAMe is poorly transported through a monolayer of Caco-2 cells and poorly absorbed by cultured rat hepatocytes (McMillan et al., (2005) J. of Pharmacy and Pharmacology, 57:599). There remains still a need to identify both the reasons why exogenous SAMe bioavailability is low and also ways in which to increase it.

US 2002/164369A discloses a composition comprising SAMe and a fatty acid. US 2002/013905A discloses a composition comprising SAMe and an unsaturated fatty acid. US 4956173A discloses a composition comprising SAMe and a stabilizer.

## SUMMARY OF THE INVENTION

The invention is defined in the claims. The present investigators have discovered that low permeability of SAMe is the primary reason why: 1) *in vivo* SAMe bioavailability is limited, 2) SAMe exhibits different absorption patterns in different regions of the GI tract and, 3) levels of SAMe metabolites are not significantly elevated after oral administration. This finding is of particular significance since, unlike overcoming liver metabolism, there are several techniques available which can alter and enhance the gastrointestinal absorption of drugs.

The present invention recognizes that SAMe permeability is low and that it is possible to increase SAMe bioavailability by utilizing factors which enhance the absorption rate of this compound.

The exemplary embodiments of the present invention relate to compositions for enhancing the absorption of S-adenosyl-L-methionine ("SAMe") or its stable salts as a means to increase SAMe bioavailability. Compositions of the invention for use *in vivo* methods provides improved bioavailability as compared to conventional non-parenteral dosage forms of SAMe.

The invention, defined in the claims, relates to oral dosage compositions of SAMe in combination with at least one absorption-enhancing technology. Absorption-enhancing technologies which act to increase absorption of a physiologically acceptable dosage of SAMe may work in a number of ways including, for example, increasing SAMe residence time in the GI tract (therefore allowing more opportunity for uptake); delivering SAMe to regions of the GI tract that exhibit increased drug absorption; adding "absorption enhancers" which increase either transcellular or paracellular transport

of drugs (including agents which directly affect tight junction opening or penetration); encapsulating SAMe in nanocarriers that deliver SAMe directly to cells; or a combination of any of such technologies which modulate absorption. An "absorption-enhancing technology" is therefore any excipient, device, mechanism, technique, method, treatment parameter or the like which either directly or indirectly affects the absorption or uptake of SAMe. Many of these technologies may be designed to exploit or optimize SAMe's inherent cationic nature at specific pH levels, for example, some may act to maintain SAMe in its cationic form which is more easily absorbed (*e.g.* in the presence of a buffer or buffering system). Accordingly, it is within the scope of the invention for the compositions of the invention to be combined with unconventional factors, such as diet (amount and/or type of food and/or beverage), dosing schedule, the presence or absence of a coating (*i.e.* uncoated SAMe may be more efficiently absorbed) as a suitable means of altering SAMe absorption. In some cases, administration of absorption-enhancing technologies prior to SAMe administration may be necessary to optimize SAMe uptake.

Site-specific delivery of SAMe to segments of the GI tract exhibiting enhanced-absorption (also known as "absorption windows") may be achieved with the use of pH-dependent coatings which target SAMe release in pH-specific regions of the GI tract.

Thus, some exemplary embodiments relate to compositions comprising pH-dependent coatings, wherein the composition of the pH-dependent coating acts to release a physiologically acceptable dosage of SAMe in segment-specific areas of the gastrointestinal (GI) tract. pH-dependent coatings allow release of SAMe in several regions along the entire GI tract in order to affect the site-specific effect of SAMe uptake and bioavailability.

Absorption of SAMe may occur throughout the entire length of the GI tract, including the stomach. By identifying regions with enhanced-absorption of SAMe, formulations targeted to these regions can be administered to ensure better control of SAMe absorption and bioavailability. pH-dependent coatings are not employed in this invention as simple enteric coatings applied to avoid degradation in the stomach. The pH-dependent coatings enable targeted delivery in the GI tract.

Thus, the invention also relates to compositions for use in methods for increasing the bioavailability of SAMe by delivering pH-dependent coated formulations of SAMe according to the claimed invention which act to release a physiologically acceptable dosage of SAMe in site-specific or pH-specific regions of the GI tract.

"Absorption enhancers", which also is meant to include agents known as "penetration enhancers", "permeability enhancers" and "promoters" typically act directly on specific aspects of the GI tract, such as paracellular transport, and affect the absorption rate of numerous drugs.

The invention defined in the claims further relates to compositions which make use of absorption enhancers to increase or promote absorption of a physiologically acceptable dosage of SAMe as a mechanism for increasing SAMe bioavailability.

Certain exemplary embodiments relate to absorption enhancers which directly modulate the activity of tight junctions. These are known as tight junction penetration agents or tight junction modulating or opening agents. Tight junctions are intercellular junctions between cells that control permeability between the cells. In this way, materials (*e.g.* APIs) cannot pass between cells but rather must be taken up by the cell and thus enables the cells to regulate what is allowed through. Tight junctions occur in many regions throughout the body including the mouth, small intestine, large intestine and colon and vary in density/tightness within different regions. Within the GI tract, tight junctions refer to the areas between adjacent endothelial cells and act to regulate the uptake of digested materials. Tight junctions are highly regulated and are one of the key elements that form the barrier between the luminal environment of the mouth and/or GI tract and the rest of the body.

The disclosure also relates to compositions which incorporate tight junction modulators to increase or promote absorption of a physiologically acceptable dosage of SAMe.

Pharmaceutical, medicinal, veterinary or nutritional preparations used for administering a physiologically acceptable dosage of SAMe include conventional solid or semi-solid tablets, pills, granules and capsules as well as controlled-release technologies such as pH-sensitive drug targeting, timed-release technologies, osmotic pumps, layered tablets, multiparticle tablets, nanocarriers or their combinations. When referring to "medicinal" preparations, purposes or treatments they are meant to include "medical foods". Medical foods are defined by the U.S. Food and Drug Administration as a food which is formulated to be consumed or administered enterally under the supervision of a physician and which is intended for the specific dietary management of a disease or condition for which distinctive nutritional requirements, based on recognized scientific principles, are established by medical evaluation.

Certain exemplary embodiments of the invention as defined in the claims further relate to compositions for oral administration of SAMe wherein SAMe is formulated in a solid or semi-solid composition which comprises one or more absorption-enhancing technology. The disclosure further provides methods of treatment wherein pharmaceutical, medicinal, veterinary or nutritional preparations of SAMe are administered in conjunction with one or more absorption-enhancing technology. Preferably, said absorption-enhancing technology is co-administered with said pharmaceutical, medicinal, veterinary or nutritional preparations of SAMe, and, even more preferably, said absorption-enhancing technology is included in said pharmaceutical, medicinal, veterinary or nutritional preparations of SAMe.

Absorption-enhancing technologies need not form part of the administered SAMe preparations and may be

administered separately. Depending on their specific mechanism of action, the chosen absorption-enhancing technology may be utilized either immediately before, after or concurrent with the SAMe formulations. Therefore, the disclosure also relates to novel methods of treating a disease or disorder in a subject in need thereof, wherein said method comprises administering a physiologically effective dosage of SAMe in combination with one or more absorption-enhancing technologies.

Certain exemplary embodiments relate to methods for increasing the bioavailability of SAMe in a subject by delivering a composition for oral administration comprising SAMe and at least one absorption-enhancing technology, wherein said absorption-enhancing technology acts either directly or indirectly to increase the absorption of a physiologically acceptable dosage of SAMe.

Diseases and/or disorders treatable with SAMe formulations of the invention are selected from the group consisting of, but not limited to, a mental or psychiatric disorder (*e.g.* psychotic/mood or non-psychotic mental disorders exemplified by depression and substance related disorders, respectively), a nervous system disease/disorder (*e.g.* a central nervous system disease exemplified by Alzheimer's), other neurological disease/disorders (*e.g.* headaches and sleep disorders), conditions associated with injury to the central nervous system, a liver disease/disorder (*e.g.* alcoholic liver disease), a cancer (*e.g.* solid and blood-borne cancers), a joint disease/disorder (*e.g.* arthritis), an inflammatory disease/disorder *(e.g.* ulcerative colitis), an autoimmune disease/disorder (*e.g.* systemic lupus erythematosis and rheumatoid arthritis), a degenerative disease/disorder (*e.g.* Amyotrophic Lateral Sclerosis), a soft-tissue disease/disorder (*e.g.* a fibromyalgia disorder), a pain disease/disorder, a genetic disorder related to hyper- or hypo-methylation, a gastrointestinal disease/disorder, a cardiovascular disease/disorder, and a disorder induced in whole or in part by oxidative or free-radical damage. Additional embodiments of the invention relate to combinations of SAMe with one or more active ingredients that are commonly prescribed or used for treatment of and/or prophylaxis of various diseases or disorders in a subject.

## BRIEF DESCRIPTION OF THE DRAWINGS

FIGURE 1A is a full-scale graph of the average plasma concentration of SAMe versus time of subjects in a pilot study who were administered 800 mg of one of three segment-specific SAMe formulations comprising coatings designed to release SAMe in the proximal GI tract (duodenum/jejunum; squares), in the distal GI tract (ileum/ascending colon; triangles) and metered throughout the entire GI tract (circles); and

FIGURE 1B is a magnified view of the graph in FIGURE 1A which better highlights the separation between the lower average plasma concentration curves;

FIGURE 2 is a graph showing the permeability of SAMe across a monolayer of Caco-2 human colonic adenocarcinoma cells alone and in the presence of EDTA or in the absence of calcium. Propranolol is included as a high permeability control;

FIGURE 3 is a graph showing the permeability of SAMe across a monolayer of Caco-2 human colonic adenocarcinoma cells alone and in the presence of various tight junction modulators;

FIGURE 4 is a graph of the average plasma concentration of SAMe as well as the SAMe metabolite, S-adenosyl homocysteine (SAH), versus time from seven subjects administered a 1600 mg dose of commercially available SAMe tosylate disulfate;

FIGURE 5 is a graph of the average plasma concentration of SAMe versus time from seven subjects each administered a 400 mg dose of an uncoated oral formulation of SAMe.

## DETAILED DESCRIPTION OF THE INVENTION

The present investigators have discovered that contrary to the general state of the art, SAMe permeability is low and also that SAMe demonstrates distinct absorption patterns within different regions of the GI tract in humans. Furthermore, they found that the level of SAMe metabolites in the blood are minimally affected upon exogenous SAMe administration, which also clearly suggests that low bioavailability of exogenous SAMe is not primarily due to extensive first pass metabolism in the liver. Finally, known tight junction modulators significantly increase the permeability of SAMe across a model monolayer of cells. The importance of these discoveries is significant since there are several techniques available which can alter and increase the gastrointestinal absorption of SAMe.

The present invention as defined in the claims recognizes that because SAMe permeability is low, it is possible to increase SAMe bioavailability by utilizing factors which enhance the absorption rate of this compound.

Some exemplary embodiments of the present invention relate to compositions that modulate and improve the absorption and bioavailability of orally administered SAMe. Related exemplary embodiments provide compositions for use in methods for therapeutic treatment of certain diseases and/or disorders and/or as nutritional supplements and/or as medical foods. Additional embodiments of the invention relate to combinations of SAMe with one or more active ingredients that are commonly prescribed or used for treatment of and/or prophylaxis of various diseases or disorders in a subject.

As used herein the term "SAMe" refers to S-adenosyl-L-methionine and its variant, S-adenosylmethionine. As shown in the structural formula presented earlier, SAMe appears as a charged species, and its ionization state varies with pH. As mentioned previously, in its solid form, SAMe is present as a salt comprised of the SAMe ion as well as one or more counter-ions. It is common to find SAMe in a stable salt form (e.g. with p-toluenesulfonic acid as the negative counter ion) alone or in combination with one or more additional salt-forming substances, for example, mineral or organic acids and/or amino acids (See US 3,893,999,). Other stable SAMe salts are described in, for example, US 5,128,249, which discloses particular stable salts of SAMe. Various morphologies of SAMe are suitable for use in the present invention. Thus, as used herein "SAMe" refers to the stable salts and amorphous forms and semicrystalline forms and crystalline forms of SAMe as well as to the ionic form of SAMe when present *in vivo.* Amorphous forms of SAMe can be employed at any particle size and particle size distribution.

Formulations for non-parenteral administration of SAMe are typically provided as solid or semi-solid products or dosage forms, such as tablets, capsules or pellets, and generally consist of a core "matrix material" which 'encapsulates' the drug as well as one or more protective coatings. "Product" or "dosage form" as used herein refers to any solid or semi-solid formulation or preparation used for non-parental administration of SAMe. Oral formulations or preparations as described herein include tablets, pastes, capsules, granules, caplets, lozenges and the like; all of which are well-known and well-documented in the art. These formulations may be administered using a clinical, pharmaceutical or veterinary dosing regimen. Oral SAMe dosage forms may also be provided as medical foods or dietary or nutritional supplements.

Non-parenterally administered SAMe formulations may be configured to enable extended release of the encapsulated SAMe. Co-owned U.S. patent application 2009/0088404, provides novel formulations of extended-release SAMe formulations. As disclosed in U.S. 2009/0088404, there are a variety of methods which can be used to prepare extended-release compositions of various types of drugs; and it is contemplated that at least one of these methodologies can be used to prepare extended-release SAMe compositions with enhanced bioavailability properties. The types of extended-release SAMe compositions that are contemplated within the scope of the present invention include osmotic dosage forms, extended-release matrices, pulsatile-release formulations and extended-release formulations coated with one or more enteric coatings all of which are described in detail in U.S. 2009/0088404.

A "physiologically effective dosage" of SAMe as used herein is meant to include an amount of SAMe which is administered under a defined dosing regimen for either clinical, pharmaceutical, medicinal, veterinary, dietary or nutritional purposes. Thus a "physiologically effective dosage" of SAMe includes a therapeutically effective dosage, a pharmaceutically acceptable dosage, a veterinary acceptable dosage, a nutraceutically acceptable dosage, a dietary acceptable dosage and a nutritionally acceptable dosage of SAMe as well as an acceptable dosage for use as a medical food and all of which are included for use in the present invention.

The relative bioavailability of SAMe formulations is determined by assessing its pharmacokinetic profile using well known techniques such as area under the curve (AUC; which is a measure of the overall exposure of a subject to SAMe in the plasma after a dose), $C_{max}$ (*i.e.* the highest concentration of SAMe in the plasma that is measured after a dose and $T_{max}$ (ie. the time after administration of a drug when the maximum plasma SAMe concentration is reached) - all of these measurements are extensively described in the art.

In some embodiments the invention relates to compositions for use in a method for treating and/or prophylaxis in a subject a disorder selected from the group consisting of, but not limited to, a mental or psychiatric disorder (*e.g.* psychotic/mood or non-psychotic mental disorders exemplified by depression and substance related disorders, respectively), a nervous system disease/disorder (*e.g.* a central nervous system disease exemplified by Alzheimer's), other neurological disease/disorders (*e.g.* headaches and sleep disorders), conditions associated with injury to the central nervous system, a liver disease/disorder (*e.g.* alcoholic liver disease), a cancer *(e.g.* solid and blood-borne cancers), a joint disease/disorder (*e.g.* arthritis), an inflammatory disease/disorder *(e.g.* ulcerative colitis), an autoimmune disease/disorder (*e.g.* systemic lupus erythematosis and rheumatoid arthritis), a degenerative disease/disorder (*e.g.* Amyotrophic Lateral Sclerosis), a soft-tissue disease/disorder *(e.g.* a fibromyalgia disorder), a pain disease/disorder, a genetic disorder related to hyper- or hypo-methylation, a gastrointestinal disease/disorder, a cardiovascular disease/disorder, and a disorder induced in whole or in part by oxidative or free-radical damage, comprising administering to said subject an exemplary composition of the present invention which enhances the absorption of a physiologically effective dosage of SAMe, whereby the enhanced-absorption provides an increase in SAMe bioavailability.

Some exemplary embodiments of the present invention relate to compositions and their use in methods for enhancing the effectiveness of a physiologically effective dosage of SAMe utilized as a dietary or nutritional supplement in a subject. Effectiveness as a dietary or nutritional supplement may be measured using one or more nutritional performance variables, such as improved concentration, memory, mood, nutritional status or liver status.

## ABSORPTION-ENHANCING TECHNOLOGIES AS A MEANS OF IMPROVING SAMe ABSORPTION AND BIOAVAILABLITY

Once it is recognized that SAMe absorption is a limiting factor in the systemic bioavailability of SAMe, it is suitable to investigate means of increasing or modulating its absorption. Any method which either directly or indirectly enhances SAMe absorption throughout the body is contemplated within the scope of this invention, including for example, increasing SAMe residence time in the GI tract thereby allowing more opportunity for uptake, delivering SAMe to targeted regions of the GI tract that exhibit increased drug absorption characteristics, incorporation of "absorption enhancers" (including "penetration enhancers" and "promoters") which increase either transcellular and/or paracellular transport of drugs (including agents which directly affect tight junctions); encapsulating SAMe in nanocarriers that deliver SAMe directly to cells; maintaining SAMe in its cationic form, modulating diet and/or dosing schedule, delivering SAMe uncoated or a combination of any of such ’technologies’ which modulate absorption. When referring to the "gastrointestinal tract" or "GI tract", it is intended to include the entire region beginning with the mouth/cheeks through to the esophagus, stomach, small intestine, large intestine and colorectal regions.

### *Mechanisms to Increase Gastric Retention Time*

Increasing SAMe gastric retention time may be achieved using, for example, gastroretentive dosage forms (GRDF) of the drug including floating, geometric, bioadhesive and swelling dosage forms which are designed to withstand peristalsis and mechanical contractility of the stomach.

### *GI Segment-Specific Targeted Formulations*

Site-specific delivery of SAMe to multiple sites along the GI tract is useful in understanding and modulating SAMe absorption since the unique environment of different segments throughout the intestinal tract can affect absorption of different drugs. In particular, drugs which show low permeability in the GI tract tend to be absorbed in specific areas along the tract. Thus, their delivery site must be controlled in order to control the absorption.

Targeted delivery sites in the GI tract include one or more of the mouth, stomach, duodenum, jejunum, ileum, colon and rectum. The pH along the GI tract varies from as low as 1 in the stomach to 8 in certain segments of the intestines. The GI tract is a highly complex environment with distinct pH zones that vary in location depending on a number of factors, including diet. Typically the pH ranges from lowest in the stomach to higher pH zones in the small and large intestine.

The large intestine is the final organ comprising the GI tract and includes the colon and rectum. The large intestine is the site for water resorption and formation of feces. Like the buccal area, blood that drains the rectum is not first transported to the liver. Therefore, absorption that takes place in the rectum (e.g., from rectal suppositories and enemas) enters the systemic circulation system without any biotransformation that may otherwise have occurred in the liver.

In addition to pH, other physiological factors such as surface area, enzymatic and transporter activity, tight junction porosity and colonic microflora influence drug absorption, and it is within the scope of the present invention to modulate one or more of these factors in any region(s) of the GI tract as a means of affecting the bioavailability of SAMe.

It is known to those skilled in the art that paracellular transport, mediated through tight junctions is higher in the proximal segments of the GI tract, exemplified by the duodenum, jejunum and ileum. Paracellular transport is much less in the distal segments, such as the colon.

Some exemplary embodiments of the present disclosure relate to novel compositions comprising pH-dependent coated SAMe, wherein the composition of the pH-dependent coating acts to release a physiologically acceptable dosage of SAMe in segment-specific areas of the gastrointestinal (GI) tract. pH-dependent coatings may be configured to enable release of SAMe in several regions along the entire GI tract in order to affect site-specific absorption and bioavailability of SAMe.

Some exemplary embodiments of the present invention relate to compositions comprising SAMe in non-enteric coated (or "uncoated") formulations. In contrast to the current general state of the art, investigators here found that SAMe can be effectively released into the stomach and give rise to elevated SAMe plasma levels and therefore, an enteric coating is not critical for achieving absorption.

*Absorption Enhancers*

The epithelial and endothelial barriers of the human body provide major obstacles for drug delivery to the systemic circulation systems and also to organs with unique environments, such as the central nervous system. Several transport routes exist in these barriers, which potentially can be exploited for enhancing drug permeability and absorption. Compared to the transcellular pathways (via transporters, adsorptive and receptor-mediated transcytosis), the paracellular flux for cells and molecules is very limited. Over the past 40 years many groups have been developing absorption or permeability enhancers. These "promoters" are generated as a means of modifying intercellular junctions and paracellular permeability.

Thus, some exemplary embodiments of the present invention as defined in the claims relate to compositions comprising a physiologically acceptable dosage of SAMe in combination with one or more "absorption enhancers". "Absorption enhancers," such as paracellular permeability enhancers (PPE) or "promoters" typically fall into the broad chemical categories of detergents or surfactants, non-surfactants (such as unsaturated cyclic ureas), fatty acids, bile acids and chelating agents. Each agent may improve absorption of orally delivered active ingredients, by one or more mechanisms exemplified by altering the rheology of the overlying mucous, fluidizing the cell membrane lipid bilayer, affecting the tight junctional complex, inhibiting enzyme or transporter activity, influencing the drug itself in some way, among others. Absorption enhancers used herein may function through a number of chemical or physical interactions including those that: (1) modulate SAMe solubility; (2) improve SAMe mucous diffusivity; (3) protect SAMe from pH, lumenal and/or brush border enzymes; (4) protect SAMe from nonspecific binding sites; and (5) improve SAMe's permeability through the mouth and/or gastrointestinal epithelium.

Examples of absorption enhancers which are suitable for use include, but are not limited to, small molecule enhancers that are commonly referred to as CPEs (chemical penetration enhancers; as listed in Table 1 below), bile salts, surfactants, phospholipids, glycerides and fatty acids, as well as peptide hormones, cytoskeletal perturbing agents, oxidants, calcium ion ($Ca^{++}$) chelators and ionophores.

Table 1. List of CPEs

| Abbreviations | Chemical Name | Category | CAS number |
|---|---|---|---|
| | | | |
| SLS | Sodium lauryl sulfate | AS | 151-21-3 |
| SDS | Sodium decyl sulfate | AS | 142-87-0 |
| SOS | Sodium octyl sulfate | AS | 142-31-4 |
| SLA | Sodium laureth sulfate | AS | 68585-34-2 |
| NLS | *N*-Lauryl sarcosinate | AS | 137-16-6 |
| CTAB | Cetyltrimethyl ammonium bromide | CS | 57-09-0 |
| DTAB | Decyltrimethyl ammonium bromide | CS | 2082-84-0 |
| BDAC | Benzyldimethyl dodecyl ammonium chloride | CS | 139-07-1 |
| TTAC | Myristyltrimethyl ammounium chloride | CS | 4574-04-3 |
| DPC | Dodecyl pyridinium chloride | CS | 104-74-05 |
| DPS | Decyldimethyl ammonio propane sulfonate | ZS | 15163-36-7 |
| MPS | Myristyldimethyl ammonio propane sulfonate | ZS | 14933-03-6 |
| PPS | Palmityldimethyl ammonio propane sulfonate | ZS | 2281-11-0 |
| CBC | ChemBetaine CAS | ZS | N/A mixture |
| CBO | ChemBetaine Oleyl | ZS | N/A mixture |
| PCC | Palmitoyl carnitine chloride | ZS | 6865-14-1 |
| IP | Nonylphenoxypolyoxyethylene | NS | 68412-54-4 |
| T20 | Polyoxyethylene sorbitan monolaurate | NS | 9005-64-5 |
| T40 | Polyoxyethylene sorbitan monopalmitate | NS | 9005-66-7 |
| SP80 | Sorbitan monooleate | NS | 1338-43-8 |

(continued)

| Abbreviations | Chemical Name | Category | CAS number |
|---|---|---|---|
| TX100 | Triton- X-100 | NS | 9002-93-1 |
| SDC | Sodium deoxycholate | BS | 302-95-4 |
| SGC | Sodium glycocholate | BS | 863-57-0 |
| CA | Cholic Acid | FA | 732163-53-8 |
| HA | Hexanoic Acid | FA | 142-91-6 |
| HPA | Heptanoic Acid | FA | 111-14-8 |
| LME | Methyl Laurate | FE | 111-82-0 |
| MIE | Isopropyl myristate | FE | 110-27-0 |
| IPP | Isopropyl myristate | FE | 142-91-6 |
| MPT | Methyl palmitate | FE | 112-39-0 |
| SDE | Dibutyl sebacate | FE | 110-40-7 |
| SOA | Sodium oleate | SS | 143-19-1 |
| UR | Urea | FM | 57-13-6 |
| LAM | Lauryl amine | FM | 124-22-1 |
| CL | Caprolactam | NR | 105-60-2 |
| MP | Methyl pyrrolidone | NR | 872-50-4 |
| OP | Octo pyrrolidone | NR | 2687-94-7 |
| MPZ | Methyl piperazine | NR | 109-01-3 |
| PPZ | Phenyl piperazine | NR | 92-54-6 |
| EDTA | Ethylenediaminetetraacetic acid | OT | 10378-23-1 |
| SS | Sodium salicylate | OT | 54-21-7 |
| CP | Carbopol 934P | OT | 9003-04-7 |
| GA | Glycyrrhetinic acid | OT | 471-53-4 |
| BL | Bromelain | OT | 9001-00-7 |
| PO | Pinene oxide | OT | 1686-14-2 |
| LM | Limonene | OT | 5989-27-5 |
| CN | Cineole | OT | 470-82-6 |
| ODD | Octyl dodecanol | OT | 5333-42-6 |
| FCH | Penchone | OT | 7787-20-4 |
| MTH | Menthone | OT | 14073-97-3 |
| TPMB | Trimethoxy propylene methyl benzene | OT | 2883-98-9 |
| AS Anionic surfactants, CS cationic surfactants, ZS zwitterionic surfactants, NS nonionic surfactants, BS bile salts, FA fatty acids, FE fatty esters, FM fatty amines, SS sodium salts of fatty acids, NR nitrogen-containing rings, OT others. | | | |

Recent advances in drug absorption research led to the discovery of an increasing number of integral membrane, adaptor, regulator and signaling proteins in tight and adherens junctions. Tight junctions are intercellular junctions between cells that form a barrier between the cells. In this way, materials (*e.g.* small molecules, proteins and drugs) cannot pass between cells but rather must be taken up by the cell and thus enables the cells to regulate what is allowed through. Tight junctions occur in many regions throughout the body including the mouth, small intestine, large intestine and colon and vary in density/tightness within different regions. Within the GI tract, tight junctions refer to the areas between

adjacent endothelial cells and act to regulate the uptake of digested materials. Tight junctions are highly regulated and are one of the key elements that form the barrier between the luminal environment of the mouth and/or GI tract and the rest of the body.

Tight junctions have three main functions: (1) to hold cells together, (2) to block the movement of integral membrane proteins between the apical and basolateral surfaces of the cell, allowing the specialized functions of each surface (for example receptor-mediated endocytosis at the apical surface and exocytosis at the basolateral surface) to be preserved (this aims to preserve the transcellular transport) and (3) to prevent the passage of molecules and ions through the space between cells and therefore materials must actually enter the cells (by diffusion or active transport) in order to pass through the tissue. This pathway provides control over what substances are allowed through.

New tight junction modulators or opening agents are currently under development, which can directly target tight or adherens junction proteins, the signaling pathways regulating junctional function, or tight junction associated lipid raft microdomains. Modulators acting directly on tight junctions include peptides derived from zonula occludens toxin, Clostridium perfringens enterotoxin, peptides selected by phage display that bind to integral membrane tight junction proteins, and lipid modulators. They can reversibly increase paracellular transport and drug delivery and have a potential to be used as pharmaceutical excipients to improve drug delivery across epithelial barriers and the blood-brain barrier. Exemplary "tight junction modulators" suitable for use include, but are not limited to, chitosan, poly(acrylic acid), cytochalasin D; caprate, spermine, taurocholate (including sodium and other salt forms) and other bile acids and/or their salts (such as cholic acid, sodium cholate or potassium cholate), as well as more recently identified agents which include peptides derived from zonula occludens toxin or Clostridium perfringens enterotoxin. Classes of tight junction modulators included herein thus include: saturated and/or unsaturated fatty acids or their corresponding carboxylate salts (e.g. C6-C24 fatty acids, or carboxylate salts thereof, especially C8-C22 fatty acids, or carboxylate salts thereof, C10-C20 fatty acids or carboxylate salts thereof, C6-, C7-, C8-, C9-, C10-, C11-, C12-, C13-, C14-, C15-, C16-, C17-, C18-, C19-, C20-, C21-, C22-fatty acids or carboxylate salts thereof), saturated and unsaturated sulfonic acids and sulfonate salts thereof (e.g. e.g. C6-C24 sulfonic acids or sulfonate salts, especially C8-C22 sulfonic acids or sulfonate salts, C10-C20 sulfonic acids or sulfonate salts, C8-, C9-, C10-, C11-, C12-, C13-, C14-, C15-, C16-, C17-, C18-, C19-, C20-, C21-, C22- sulfonic acids or sulfonate salts); zwitterionic surfactants (e.g. 3-(N,N-Dimethylpalmitylammonio)propanesulfonate, decyldimethyl ammonio propane sulfonate, myistyldimethyl ammonio propoane sulfonate, cocamidopropyl hydroxysultaine (ChemBetaine® CAS), oleyl betaine (ChemBetaine® Oleyl), or palmitoyl carnitine chloride); fatty amines (e.g. C6-C24 fatty amines, especially C8-C22 fatty amines, C10-C20 fatty amines, C6-, C7-, C8-, C9-, C10-, C11-, C12-, C13-, C14-, C15-, C16-, C17-, C18-, C19-, C20-, C21-, C22-fatty amines), as well as other organic acids (e.g. tartaric acid) and cyclodextrins (e.g. alpha-cyclodextrin, beta-cyclodextrin, or gamma-cyclodextrin). Exemplary fatty acids that may be used include hexanoic, heptanoic, capric, lauric acid, myristic acid, palmitic acid, stearic acid, arachidic acid, myristoleic acid, palmitoleic acid, sapienic acid, oleic acid, linoleic acid, $\alpha$-linolenic acid, arachidonic acid, eicosapentaenoic acid, erucic acid, docosahexaenoic acid. Exemplary carboxylate salts that may be used include sodium or potasium captrate, caprylate, laurate, myristate, palmitate, stearate, arachidate, myristoleate, palmitoleate, sapienate, oleate, linoleate, $\alpha$-linolenate, arachidonate, eicosapentaenoate, erucate, docosahexaenoate. Specific carboxylate salts include sodium caprate, sodium caprylate, and sodium laurate. Specific fatty amines that may be used include lauryl amine (N-dodecylamine), decylamine, nonylamine, octylamine, heptylamine or hexylamine. Exemplary sulfonic acids that may be used include octane sulfonic acid, decane sulfonic acid (e.g. sodium 1-decanesulfonate), dodecane sulfonic acid, tetradecane sulfonic acid, hexadecane sulfonic acid, octadecane sulfonic acid, eicosane sulfonic acid, docosane sulfonic acid or tetracosane sulfonic acid. Specific sulfonic acids that may be mentioned include dioctyl sodium sulfosuccinate.

Thus the invention as defined in the claims specifically relates also to compositions comprising a physiologically acceptable dosage of SAMe and at least one tight junction modulator. In preferred embodiments, said tight junction modulator is co-formulated with the physiologically acceptable dosage of SAMe.

Tight junction modulators may be particularly effective in improving modified release dosage forms targeting more distal segments of the GI tract. The porosity of tight junctions is tighter in distal segments such as the ileum and colon, compared to, for example, the duodenum (*i.e.* tight junctions of the duodenum are more porous than those of the lower GI segments.) In addition, transit time in the upper GI tract is faster than in the lower GI tract. The combinations of less porous tight junctions coupled with the slower transit time in the lower segments suggest that the use of tight junction modulators in the lower GI tract may be more impactful on a relative basis. This effect could be expected to extend to SAME delivery in the colon and in the case of suppository formulation, to the rectum.

Improved buccal delivery of SAMe is also considered practical using formulations of the invention comprising one or more tight junction modulators considering the presence of tight junctions in the mouth. Thus non-parenteral formulations of the invention are meant to include those which target buccal, upper and lower intestinal regions including the colon and rectum.

It would also be possible to use a lower GI targeted modulator-enhanced component in combination with an upper GI-targeted conventional or modulator-enhanced component to create a modified release dosage form with enhanced absorption over an extended period of time.

Thus the invention specifically relates to compositions for buccal delivery comprising a physiologically acceptable dosage of SAMe and at least one tight junction modulator.

In some embodiments the composition is administered as a buccal dosage form. In other embodiments the composition is administered as a suppository.

Preferably, the suitability of a particular "absorption enhancer", including "tight junction modulators," will be identified *in vitro* by use of SAMe cellular permeability studies. Most relevant cell lines will suffice for such *in vitro* experimentation including, but not limited to, Caco-2 cells (as described in Example 3). In addition, the use of references in the art may also provide insight into potentially suitable "absorption enhancers" or "tight junction modulators" for use in the present invention.

*Nanocarriers to Increase Delivery of SAMe*

Encapsulating SAMe into nano-sized carriers which are suitable for use in non-parenteral administration of SAMe (*e.g.* nanoparticles and colloidal systems) may result in increased delivery to the cells. Several approaches have been described that appear to increase transcellular intestinal absorption without damaging the epithelium. These approaches can be categorized into methods that stabilize the drug, increase drug solubility or alter its characteristics to improve transcellular permeability. Various colloidal systems which may be suitable for enhancing the absorption of SAMe are exemplified by submicron emulsions, polymeric nanoparticles, microparticles, and the like. There are various physicochemical factors governing gastrointestinal uptake of such systems including size, size distribution, consistency, hydrophobicity and surface properties which may be modulated in order to enhance SAMe cellular uptake.

**DOSING WITH FORMULATIONS EXHIBITING ENHANCED-ABSORPTION AND BIOAVAILABLITY OF SAMe**

In some embodiments the enhanced-absorption SAMe formulations of the present invention relate to enhanced nutritional support, or dietary supplement health improvements including, but not limited to, mood improvement, joint health and liver function. In some exemplary embodiments the disorder is related to the dietary management of a disease through additional supplementation of SAMe which cannot be reached through diet (*e.g.* a "medical food".)

Some exemplary embodiments of the invention relate to compositions for use in a method for treating and/or prophylaxis in a subject a disease or disorder selected from the group consisting of, but not limited to, a mental or psychiatric disorder (*e.g*. psychotic/mood or non-psychotic mental disorders exemplified by depression and substance related disorders, respectively), a nervous system disease/disorder (*e.g.* a central nervous system disease exemplified by Alzheimer's), other neurological disease/disorders (*e.g*. headaches and sleep disorders), conditions associated with injury to the central nervous system, a liver disease/disorder (*e.g.* alcoholic liver disease), a cancer (*e.g.* solid and blood-borne cancers), a joint disease/disorder *(e.g.* arthritis), an inflammatory disease/disorder (*e.g.* ulcerative colitis), an autoimmune disease/disorder (*e.g*. systemic lupus erythematosis and rheumatoid arthritis), a degenerative disease/disorder (*e.g.* Amyotrophic Lateral Sclerosis), a soft-tissue disease/disorder (*e.g.* a fibromyalgia disorder), a pain disease/disorder, a genetic disorder related to hyper- or hypo-methylation, a gastrointestinal disease/disorder, a cardiovascular disease/disorder, and a disorder induced in whole or in part by oxidative or free-radical damage, comprising administering to said subject an exemplary composition of the present invention which enhances the absorption and bioavailability of a physiologically effective amount of exogenous SAMe.

Some embodiments of the present invention relate to compositions for therapeutic use for treatment of a mental or psychiatric disorder selected from the group consisting of anxiety disorders, depressive disorders, eating disorders, bipolar disorder, abuse disorders, dependence disorders, Axis II disorders, and psychosis. In some exemplary embodiments, the mental or psychiatric disorder is an anxiety disorder selected from the group consisting of generalized anxiety disorder, posttraumatic stress disorder, social anxiety disorder, panic disorder, Schizophrenia and obsessive compulsive disorder. In some exemplary embodiments, the mental or psychiatric disorder is a depressive disorder selected from the group consisting of major depressive disorder, multi-infarct dementia, minor depression, postpartum or late-life depression (and the like), Parkinson's depression, HIV-associated depression, brief recurrent depression, dysthymia or depression NOS (Not Otherwise Specified). In some exemplary embodiments, the mental or psychiatric disorder is an eating disorder selected from the group consisting of bulimia nervosa, anorexia nervosa, binge eating disorder, obesity, or eating disorder NOS. In some exemplary embodiments, the mental or psychiatric disorder is bipolar disorder, an abuse disorder or a dependence disorder, including abuse of, or dependence on, alcohol, nicotine, cocaine, codeine, oxycodone, hydrocodone or other opiates. In some exemplary embodiments, the mental or psychiatric disorder is an Axis II disorder selected from borderline personality disorder.

In some exemplary embodiments, the disorder is a nervous system disorder, including a central nervous system (CNS) disorder such as Parkinson's disease, Alzheimer's disease, Angelman Syndrome (genetic disorder), Multiple Sclerosis (MS) and pre-dementia and/or cognitive impairment.

In some exemplary embodiments, the disorder is a comorbid disorder, such as comorbid depression arising in a subject who is undergoing treatment for one or more diseases or disorders such as but not limited to, cancer, Parkinson's and HIV. In certain embodiments the comorbid disorder is caused by one or more therapies being utilized to treat said one or more diseases or disorders.

In some exemplary embodiments, the disorder is a result of an injury to the CNS such as spinal cord injury or brain damage, memory loss, cognitive impairment and/or learning disability.

In some exemplary embodiments, the disorder is a liver disorder selected from the group consisting of alcoholic liver disease, fatty liver disease (non-alcoholic) hepatitis (both viral and non-viral), liver cancer, oxidative liver disease, HISS-dependent insulin resistance, cholestasis and cirrhosis.

In some exemplary embodiments, the disorder is a cancer selected from the group consisting of cancers occurring in one or more of the liver, colon, rectum, ovaries, urethra, testicles, bladder, breast, stomach, esophagus, pancreas, head and neck, lung, blood, skin (such as actinic keratosis, basal cell cancer, superficial basal cell cancer, squamous cell cancer, and melanoma) and adenocarcinomas.

In some exemplary embodiments, the disorder is a joint disorder such as, for example, arthritis and osteoarthritis.

In some exemplary embodiments, the disorder is an inflammatory disorder selected from the group comprising systemic lupus erythematosis, Reye's syndrome, rheumatic fever, allergic rhinitis, myasthenia gravis, temporal arteritis, vasculitis, psoriasis, atopic dermatitis, rosacea, eczema, alopecia universalis, scleroderma, pemphigus, contact dermatitis, ankylosing spondylitis, dermatomyositis, polymyositis, celiac sprue, Guillain-Barré syndrome, multi-infarct dementia, post-cerebral vascular accident reperfusion damage, Addison's disease, Hashimoto's thyroiditis, asthma, upper respiratory inflammation symptoms, chronic bronchitis, atherosclerosis, pernicious anemia, autoimmune hepatitis, prostatitis, pelvic inflammatory disease, Goodpasture's syndrome, Wegener's granulomatosis, chronic nephritis, Sjogrens syndrome, or allergic conjunctivitis.

In some exemplary embodiments, the disorder is a gastrointestinal disorder such as inflammatory bowel disease (IBD), Crohn's disease or ulcerative colitis (UC).

In some exemplary embodiments, the disorder is a soft tissue disease such as fibromyalgia.

In some exemplary embodiments, the disorder is a pain disorder such as fibromyalgia, chronic headaches, shingles, reflex sympathetic dystrophy and polyneuropathy.

In some exemplary embodiments, the disorder is a cardiovascular disorder which is related to hyper- or hypohomocysteinemia such as coronary heart disease, stroke, peripheral vascular disease and atherosclerotic disease.

In some exemplary embodiments, the disorder is related to a genetic or medical condition related to a deficiency of the methylation pathway such as methylenetetrahydrofolate reductase deficiency.

In some exemplary embodiments, the etiology of the disorder may include oxidative or free-radical damage, and is selected from the group comprising chronic fatigue syndrome, temporal arteritis, vasculitis, multi-infarct dementia, chronic emphysema, or chronic nephritis.

Among the advantages provided by enhanced-absorption SAMe formulations of the invention, included are the convenience and concomitant improved subject compliance due to reduced daily dosing, an improved side-effect profile (such as decreased stomach irritation and potentially decreased tendency to induce mania in manic depressive subjects or subjects at risk for manic episodes) and other side effects associate with or caused by the relatively high doses of SAMe (typically about 400 to about 3200 mg SAMe ion/day, more typically about 800 to about 1600 mg SAMe ion/day) necessary to achieve a desired effect.

As used herein, the term "desired effect" includes a "therapeutic effect", "pharmaceutical effect", "dietary effect" (*e.g.* for use as a medical food), "nutraceutical effect" and "nutritional effect". Thus, a "desired effect" includes ameliorating at least one symptom of a physiological disorder or disease state in a subject, or improving at least one performance variable (such as improved concentration, memory, mood, nutrition status or liver status) when used as a nutritional supplement in a subject. The "desired effect" may be achieved through nutritional supplementation using SAMe formulations of the invention or through administration using a clinical, pharmaceutical or veterinary dosing regimen of SAMe formulations of the invention.

Suitable subjects for dosing according to the methods and compositions of the invention include warm-blooded mammals such as humans, domestic or exotic animals or livestock; domesticated avian subjects such as chickens and ducks; and laboratory animals suitable for research use. When used for treating a disease or disorder in a subject, various symptoms of specific physiological disorders and disease states are contemplated as being treatable within the context of the present invention and details of which are set forth below. However, it is to be recognized that the understanding of various disease states by those of skill in the art is not static and this is the same for performance variables related to nutritional supplementation. Thus, though the description above is intended to be illustrative of the various disorders, disease states, symptoms or performance variables that may be treated using the enhanced-absorption SAMe formulations according to the present invention, a person skilled in these arts will be expected to apply such knowledge.

*Dosing with Multiple Dosing Units*

Some exemplary embodiments of the present invention relate to compositons for use in treatment of and/or prophylaxis of one or more diseases in a subject, wherein the treatment of and/or prophylaxis of one or more diseases and/or disorders comprises administering to the subject an absorption-enhanced formulation comprising a physiologically acceptable dosage of S-adenosyl methionine (SAMe), or a proprietary salt thereof.
Some other exemplary embodiments of the present invention relate to SAMe nutritional supplements and/or dietary supplements for improvement of one or more nutritional performance variables in a subject, wherein the nutritional performance variables are one or more of concentration, memory, mood, nutritional status and liver status, and wherein an absorption enhanced formulation comprising a physiologically acceptable dosage of S-adenosyl methionine (SAMe), or other proprietary SAMe salts thereof, is administered to a subject.
In some exemplary embodiments, the absorption-enhanced SAMe may be divided between multiple daily doses. Multiple daily doses need not be identical and may comprise one or more dosage forms in combination. In some exemplary embodiments, the enhanced-absorption SAMe may be divided into two or more daily doses. Each dose may be administered as a single dosage unit exemplified by, a single tablet, capsule or caplet, or alternatively may be divided into multiple dosage units. In some embodiments, a twice-daily dose of from about 100 to about 1600 mg of SAMe ion per dose may be divided into one to four dosage units of from about 100 to about 800 mg of SAMe ion per unit. In each case, the form of the dosage unit may be a capsule, a tablet, a caplet (single or multi-compartment) or an extended release dosage unit and the like. In some embodiments, the absorption-enhancer and SAMe are provided in a oral dosage form wherein separate compartments of the oral dosage form contain either the absorption-enhancing agent or SAMe. In other embodiments the absorption-enhancing technology is administered separately from the SAMe dosage form. Preferably, the oral dosage form is a tablet, capsule or gel-capsule.
Conventional SAMe dosing generally administers up to 1600 mg of SAMe ion per day bi-daily (BID) in order to achieve maximum activity of the drug. Tablets are most often available commercially in 200 mg and 400 mg doses SAMe ion which require subjects to ingest 4-8 tablets per day. This is inconvenient with respect to the amount of time needed as well as the potential error in consistent dosing (*i.e.* if a dose is missed). The present invention has identified novel compositions and methods which reduce the effective dose of SAMe (*i.e.* reduce the number of tablets necessary in a day to achieve the same or better efficacy as compared to conventional dosing regimens) and/or eliminate the need to dose bi-daily. By improving SAMe absorption, a new method of SAMe therapy is available which lowers the amount of SAMe dose required to elicit an effective response by providing compositions comprising one or more absorption-enhancing technologies. These exemplary "low dose" formulations may provide a lower daily pill count which is beneficial to those taking SAMe as it will reduce the time, cost and inconvenience of self-administering large doses.
Some exemplary embodiments relate to administration of the selected physiologically acceptable dosage on a once-a-day basis. In some embodiments, the once-a-day dose may be administered in a single dosage unit exemplified by, a single tablet, capsule, or caplet. In other exemplary embodiments, the single dose may be administered as multiple tablets, capsules or caplets taken at one time. In some embodiments, for instance, a dosage of about 400 to 3200 mg of SAMe per day may be divided into two, three, four or more tablets, capsules or caplets of about 50 to 2000, preferably about 100 to 1600 mg of SAMe per unit. In some preferred embodiments, the daily dose may comprise two, three or four units (e.g. tablets, capsules or caplets) of about 100 to 800 mg of SAMe ion per unit. Suitable dosage regimens included are: four units of about 50-400 mg of SAMe ion per unit, e.g. 50, 100, 150, 200, 250, 300, 350 or 400 mg SAMe ion per unit; three units of about 50-1000 mg of SAMe ion per unit, e.g. 50, 100, 150, 200, 250, 300, 350, 400, 450, 500, 550, 600, 650, 700, 750, 800, 850, 900, 950 or 1,000 mg of SAMe ion per unit; two units of about 50-1600 mg of SAMe ion per unit, e.g. about 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550 or 1600 mg of SAMe ion per unit.
Some exemplary embodiments of the present invention relate to "low-dose" SAMe compositions. By increasing the bioavailability of exogenous SAMe, the daily administered dose of SAMe may be substantially lowered by administration of compositions with improved SAMe absorption. These exemplary "low-dose" treatments may enable a lower daily pill count.

*Fed vs. Fasting Dose*

In some embodiments of the invention, it may be advantageous to ensure that the subject is either fed or fasted (e.g. overnight for at least about 6, especially about 8, hours). It is considered that food administered at the same time, immediately (*i.e.* less than about 30, especially less than about 15 minutes) before or soon (e.g. less than about 10 minutes) after the absorption enhanced SAMe formulation of the invention is administered to the subject may increase or decrease the rate of gastric emptying and thus affect the rate of uptake of SAMe from the formulation. Thus, in some embodiments, the disclosure contemplates administering the absorption enhanced SAMe formulation of the invention with food, wherein food is ingested either before or during SAMe treatment.

COMBINATIONS OF SAMe WITH OTHER ACTIVE INGREDIENTS

Some exemplary embodiments of the present invention relate to combinations of the claimed SAMe compositions with one or more active ingredients that are commonly prescribed or used for treating and/or prophylaxis in a subject a disease or disorder selected from the group consisting of, but not limited to, a mental or psychiatric disorder (*e.g.* psychotic or non-psychotic mental disorders such as depression and substance abuse disorders, respectively), a nervous system disease/disorder (*e.g.* a central nervous system disease such as Alzheimer's), other neurological disease/disorders (*e.g.* headaches and sleep disorders), conditions associated with injury to the central nervous system, a liver disease/disorder *(e.g.* alcoholic liver disease), a cancer (*e.g.* solid and blood-borne cancers), a joint disease/disorder *(e.g.* arthritis), an inflammatory disease/disorder (*e.g.* ulcerative colitis), an autoimmune disease/disorder (*e.g.* systemic lupus erythematosis and rheumatoid arthritis), a degenerative disease/disorder (*e.g*. Amyotrophic Lateral Sclerosis), a soft-tissue disease/disorder (*e.g.* a fibromyalgia disorder), a pain disease/disorder, a genetic disorder related to hyper or hypo methylation, a gastrointestinal disease/disorder, a cardiovascular disease/disorder, and a disorder induced in whole or in part by oxidative or free-radical damage, comprising administering to said subject an exemplary composition of the present invention which enhances the absorption and bioavailability of a physiologically effective amount of exogenous SAMe.

In some exemplary embodiments of the present invention relate to combinations of the claimed SAMe compositions with one or more active ingredients that are commonly prescribed or used for treatment of and/or prophylaxis of mental or psychiatric disorders in a subject include, but are not limited to, tricyclic antidepressants (TCAs), tetracyclic antidepressants, aminoketones, phenylpiperazines, selective serotonin reuptake inhibitors (SSRIs), monoamine oxidase inhibitors (MAOIs), serotonin-norepinephrine reuptake inhibitors (SNRIs), norepinephrine-serotonin reuptake inhibitors (NSRIs), dopamine reuptake inhibitors, norepinephrine-dopamine reuptake inhibitors, norepinephrine reuptake inhibitors, selective serotonin reuptake enhancers, noradrenergic and serotonin specific antidepressants, substance P receptor antagonists, neurokinin receptor antagonists such as saredutant, corticotrophin release factor antagonists such as mifepristone, atypical antipsychotics such as aripiparazole, commonly used antidepressant augmenters such as lithium or triple reuptake inhibitors,.

Some exemplary embodiments of the present invention relate to combinations of the claimed SAMe compositions with one or more device therapies that are commonly prescribed or used for treatment of and/or prophylaxis of mental or psychiatric disorders in a subject include, but not limited to ECT (electro convulsive therapy) and electric shock therapy.

In some exemplary embodiments of the present invention relate to combinations of the claimed SAMe compositions with one or more active ingredients that are commonly prescribed or used for treatment of and/or prophylaxis of a nervous system disease/disorder in a subject include, but are not limited to anticonvulsants such as pregabalin, $\alpha$-amino-3-hydroxy-5-methyl-4-isoxazolepropionic acid (AMPA) receptor antagonists, methylphosphonate (NMPA) receptor antagonists, histamine receptor antagonists, nitric oxide (NO) modulators, glutamate receptor antagonists, acetylcholinesterase inhibitors, dopamine agonists, N-methyl-d-aspartate (NMDA) receptor antagonists such as memantine, cholinesterase inhibitors such as donepezil, neuroprotectants, nootropic agents, CNS modulators, antiamyloidogenics.

In some exemplary embodiments of the present invention relate to combinations of the claimed SAMe compositions with one or more active ingredients that are commonly prescribed or used for treatment of and/or prophylaxis of a liver disorder in a subject include, but are not limited to, antiviral medication such as alpha interferon, ribavirin, lamivudine, steroids, antibiotics and zinc acetate.

In some exemplary embodiments of the present invention relate to combinations of the claimed SAMe compositions with one or more active ingredients that are commonly prescribed or used for treatment of and/or prophylaxis of a cancer in a subject include, but are not limited to, chemotherapeutic agents, drug resistance modulators, monoclonal antibodies, cytokines
(*e.g.* interferons and interleukins), immunocytokines, growth factors, chemoprotectants, vaccines and other biological response modifiers.

In some exemplary embodiments of the present invention relate to combinations of the claimed SAMe compositions with one or more active ingredients that are commonly prescribed or used for treatment of and/or prophylaxis of a joint or inflammatory disease/disorder in a subject include, but are not limited to, analgesics, non-steroidal antiinflammatory drug compounds (NSAID), disease-modifying antirheumatic drugs (DMARDs), corticosteroids, anakinra (an interleukin-1 receptor antagonist), COX-2 inhibition, gamma-aminobutyric acid-B (GABAB) receptor agonists, such as baclofen, GABAA potentiating drugs, such as the benzodiazepines tumor necrosis factor (TNF)-inhibiting drugs, and other drugs that modify the immune response (immunosuppressive drugs).

In some exemplary embodiments of the present invention relate to combinations of the claimed SAMe compositions with one or more active ingredients that are commonly prescribed or used for treatment of and/or prophylaxis of an autoimmune disease/disorder in a subject include, but are not limited to, DMARDs, corticosteroids, anakinra (an interleukin-1 receptor antagonist), TNF-inhibiting drugs, and other drugs that modify the immune response (immunosuppressive drugs).

In some exemplary embodiments of the present invention relate to combinations of the claimed SAMe compositions with one or more active ingredients that are commonly prescribed or used for treatment of and/or prophylaxis of a degenerative disease/disorder in a subject include, but are not limited to, NSAIDs, COX-2 inhibition, GABAB receptor agonists, such as baclofen, and GABAA potentiating drugs, such as the benzodiazepines.

In some exemplary embodiments of the present invention relate to combinations of the claimed SAMe compositions with one or more active ingredients that are commonly prescribed or used for treatment of and/or prophylaxis of a soft tissue disease/disorder in a subject include, but are not limited to, milnacipram, pregabalin, SNRIs, NSRIs, muscle relaxers, sedatives, painkillers, and NSAIDs.

In some exemplary embodiments of the present invention relate to combinations of the claimed SAMe compositions with one or more active ingredients that are commonly prescribed or used for treatment of and/or prophylaxis of a genetic disease/disorder related to hyper or hypo methylation in a subject include, but are not limited to methionine, MTA (5'-deoxy-5'-(methylthio) adenosine) and other SAMe metabolites.

In some exemplary embodiments of the present invention relate to combinations of the claimed SAMe compositions with one or more active ingredients that are commonly prescribed or used for treatment of and/or prophylaxis of a gastrointestinal disease/disorder in a subject include, but are not limited to, 5-Aminosalicylic acid (5-ASA) medications, Corticosteroids (prednisone), immunomodulatory medications such as Azathioprine (Immuran), 6-Mercaptopurine (6-MP), Methotrexate and Cyclosporine (Sandimmune), commonly used antibiotics such as Metronidazole (Flagyl) and Ciprofloxacin (Cipro) and biologic agents such as Infliximab (Remicade).

In some exemplary embodiments of the present invention relate to combinations of the claimed SAMe compositions with one or more active ingredients that are commonly prescribed or used for treatment of and/or prophylaxis of a cardiovascular disease/disorder in a subject include, but are not limited to, statins, angiotensin-converting enzyme (ACE) inhibitors, ASA, SAMe break down products such as methionine, MTA and folate, cardioprotectants, vasoprotectants, coagulation inhibitors.

In some exemplary embodiments of the present invention relate to combinations of the claimed SAMe compositions with one or more active ingredients that are commonly prescribed or used for treatment of and/or prophylaxis of a disorder induced in whole or in part by oxidative or free-radical damage including, but are not limited to, antioxidants such as Vitamin A, Vitamin C, Vitamin E, polyphenols, flavonoids, selenium, carotenoids.

In some exemplary embodiments of the present invention relate to combinations of the claimed SAMe compositions with one or more active ingredients that are commonly prescribed or used for treatment of and/or prophylaxis of a disorder induced in whole or in part by damage to the central nervous system such as brain injury or spinal cord injury including, but not limited to, neuroprotectants, nootropic agents, CNS modulators, analgesics, muscle relaxants, apoptosis inhibitors, bone modulators, antioxidants.

In some exemplary embodiments of the present invention relate to combinations of the claimed SAMe compositions with methionine, MTA, folate, vitamin B6 and/or B12 as they are each correlated with lowering homocysteine production. Therefore, it is considered that combining SAMe with methionine, MTA, folate, vitamin B6 and/or B12 may result in increased supplementation of SAMe by enhancing the body's natural ability to make SAMe while at the same time supplementing SAMe with exogenous SAMe exhibiting enhanced absorption and improved bioavailability. As used herein the term "folate" refers to vitamin B9 in all of its natural and synthetic forms including, but not limited to, folic acid, tetrahydrofolate and L-methylfolate.

In some embodiments, an exemplary enhanced-absorption SAMe dosage form according to the invention may be included in a kit with a separate dosage form containing at least one other active ingredient, exemplified by one or more compounds suitable for the treatment of or commonly prescribed or used for the treating and/or prophylaxis in a subject a disease or disorder selected from the group consisting of, but not limited to, a mental or psychiatric disorder (*e.g.* psychotic/mood or non-psychotic mental disorders such as depression and substance related disorders, respectively), a nervous system disease/disorder (*e.g.* a central nervous system disease such as Alzheimer's), other neurological disease/disorders (*e.g.* headaches and sleep disorders), conditions associated with injury to the central nervous system, a liver disease/disorder *(e.g.* alcoholic liver disease), a cancer *(e.g.* solid and blood-borne cancers), a joint disease/disorder (*e.g.* arthritis), an inflammatory disease/disorder (*e.g.* ulcerative colitis), an autoimmune disease/disorder (*e.g.* systemic lupus erythematosis and rheumatoid arthritis), a degenerative disease/disorder *(e.g.* Amyotrophic Lateral Sclerosis), a soft-tissue disease/disorder *(e.g.* a fibromyalgia disorder), a pain disease/disorder, a genetic disorder related to hyper or hypo methylation, a gastrointestinal disease/disorder, a cardiovascular disease/disorder, and a disorder induced in whole or in part by oxidative or free-radical damage, comprising administering to said subject an exemplary composition of the present invention which improves the absorption of a physiologically effective amount of exogenous SAMe.

In addition to combinations of SAMe with the one or more additional ingredients exemplified above or methionine, MTA, folate, vitamin B6 and/or B12, administration of the exemplary enhanced-absorption SAMe formulations of the invention may also augment the effects of other drugs or nutritional supplements being taken by the subject. Thus, some

exemplary embodiments of the present invention relate to combinations of SAMe with drugs or nutritional compounds already employed for treating other diseases for increasing the activity of said drugs or nutritional compounds.

The present invention is further described by the following examples. These examples, while illustrating certain specific aspects of the invention, should not be considered to limit or circumscribe the scope of the disclosed invention.

**EXAMPLES**

Reference Example 1

Altered SAMe Coating Compositions Result in GI Segment-Specific SAMe Absorption

In order to better understand the absorption characteristics of SAMe *in vivo,* standard, uncoated tablets comprising SAMe were first generated and then covered with a segment-specific coating targeting one of three distinct regions of the GI tract.

The uncoated SAMe tablets comprising microcrystalline cellulose, croscarmellose, colloidal silicon dioxide and magnesium stearate were made using standard procedures known to those skilled in these arts. In order to improve the compressibility of the composition, SAMe powder was granulated using a dry compaction process. Each excipient was split between the intra-granular and extra-granular phases. The final tableting mixture was compressed using a rotary tablet press fitted with elongated oval tooling at one station and the remaining stations blocked off. The relative ambient humidity was maintained at around 30% or less and ambient temperature was controlled between 20 and 30°C throughout the process. The granules used in this formulation had good flow properties and demonstrated no sticking or picking during compression.

In order to target SAMe release in the proximal GI tract (duodenum & jejunum) a commonly used Eudragit® coating known to dissolve above a pH of about 5.5, was applied to the SAMe tablets (EUDRAGIT is a registered trademark of Rohm GmbH; Darmstadt, Germany). In an attempt to improve the surface properties of these tablets prior to applying the pH-dependent coating, a commercially available seal coat was first applied.

A second formulation comprising a second commercially available Eudragit® coating was utilized to deliver SAMe to the distal GI tract (ileum/ascending colon) as it dissolves at a pH above about 7.0. As above, this formulation was first prepared with a commercially available seal coat.

Finally, a rate controlling coating used to provide metered SAMe release throughout the entire GI tract was applied to uncoated SAMe core tablets by a Contract Research Organization.

As seen in Figure 1A, delivery of SAMe is achieved in all three regions of the GI tract and each site results in a unique pharmacokinetic profile of SAMe with different Cmax and Tmax values (Figure 1B). In the case of the pH 5.5 and pH 7.0 coated tablets, the expected time of un-coating is a function of the pH sensitivity of the coating together with the expected transit or arrival time for the targeted segments. The Tmax observed experimentally corresponded to that anticipated time. In the case of the rate-controlling coating which is a non-dissolving coating, there is no seal coating, the Tmax corresponds to maximal drug delivery in the colon.

The three formulations therefore were confirmed to show targeted delivery at three distinct sites within the GI tract corresponding to proximal, distal and extended GI segments as judged by their Tmax. The core of the three products and the dose applied was identical but the amount delivered as indicated by the Cmax and the AUC was significantly different. The relative bioavailability correlates with the tight junction porosity of the targeted segment.

Example 2

*In Vivo* Analysis of Absorption-Enhancing Agents

Use of absorption enhancers as a means to increase the absorption and thus bioavailability of a novel preparation of SAMe is achieved by either co-formulating SAMe with one or more absorption enhancers or co-administering SAMe with one or more absorption-enhancing agents. Co-administration may not necessarily be at the same time as it may be more efficacious to administer said absorption enhancers within a reasonable time either before or after administration of said proprietary preparation of SAMe.

Identification of suitable absorption enhancers may be found in the art or may be achieved *in vivo. In vivo* activity of compositions comprising SAMe and one or more absorption enhancing agent may be measured after administration into an animal model. Preferably, the animal model comprises a pharmacokinetic (PK) model wherein candidate formulations are administered using pharmacologically effective doses to non-rodent animals (for example dog, pig, mini-pig, or primate) and blood, urine, cerebrospinal fluid (CSF) or other appropriate biological fluid is removed at periodic intervals. The biological fluid is tested for active compound in order to construct concentration vs. time profiles. These data are analyzed and pharmacokinetic parameters are calculated in order to assess in vivo pharmacokinetic activity.

The most common pharmacokinetic parameters analyzed in such models are Cmax, Tmax, and area under the curve (AUC).

Alternatively, or in addition to the PK model, one can identify suitable absorption enhancers using efficacy as a measurement, through the use of non-rodent models for liver disease or osteoarthritis as an example.

Plasma and urine markers include measuring markers suitable for each disease/disorder.

Changes in gene expression include serial analysis of gene expression (genomics) and changes in protein expression (proteomics) or changes in metabolite levels (metabolomics).

Example 3

*In Vitro* Screening of Absorption-Enhancing Agents

In addition to above, identification of suitable absorption enhancers may also be achieved using simple, standard *in vitro* screening assays. In the present invention, permeability of SAMe across Caco-2 cell monolayers treated with an absorption enhancer is used to identify agents which increase the amount of SAMe absorbed by said Caco-2 cells in comparison to untreated Caco-2 cell monolayers. The Caco-2 cell line is derived from a human colorectal carcinoma and is widely used for *in vitro* cell culture models for the study of gastrointestinal drug absorption (Stewart, B., (1995) *Pharm. Res.* 12:693). In these models, pure cell lines are grown on a semi-permeable membrane. Drug formulations are placed on the apical or basolateral side of the cell monolayer and transport is determined via measurement of drug concentrations on the other side of the membrane.

The Caco-2 cell line utilized here was from the American Type Culture Collection (ATCC). Caco-2 cells are grown in Dulbecco's modified Eagle's medium (DMEM, Gibco) supplemented with 20% FBS (fetal bovine serum, Gibco), 100 uM non-essential amino acids (NEAA, Gibco) and 2mM L-glutamine (Gibco). A Beckton Dickinson BIOCOAT® HTS Caco-2 Assay System Kit is used resulting in $6.6 \times 10^5$ cells/$cm^2$ seeding density (BIOCOAT is a registered trademark of Collaborative Biomedical Products, Inc., Bedford, Massachusetts, USA). The cells used in transport studies are grown for 3 days before the experiments. The culturing conditions are 37°C in an atmosphere of 5% $CO_2$ and 100% humidity.

For permeability across Caco-2 cell monolayers, the transport medium used was Hank's Buffered Salt Solution (HBSS; purchased from Gibco) containing D-glucose, and HEPES pH adjusted to 7.4. A 2mM aqueous solution of either SAMe tosylate disulfate or SAMe 1,4 butanedisulfonate was added on the apical or basolateral side according to the manufacturer's procedure for the Caco-2 kit. Samples were measured after 120 minute incubation by liquid chromatography-mass spectrometry (LC/MS). The integrity of the monolayers was monitored using Lucifer Yellow Assay. As an example, the effect of the absorption enhancer (and specifically a tight junction opening agent), EDTA (2mM in wells), as well as calcium-free medium on Caco-2 permeability of SAMe is compared to absorption of SAMe on its own. Propranolol is a high permeability marker and was utilized as a positive control for a readily absorbed molecule.

The results in Table 2 below as well as those depicted in Figure 2 show that SAMe absorption on its own is low for both salts as evidenced by a low apparent permeability coefficients (Papp = $0.41 \times 10^{-6}$ and $0.50 \times 10^{-6}$ cm $s^{-1}$ in apical to basolateral and basolateral to apical directions, respectively for SAMe disulfate tosylate; and Papp = $0.50 \times 10^{-6}$ and $0.60 \times 10^{-6}$ cm $s^{-1}$ in apical to basolateral and basolateral to apical directions, respectively for SAMe 1,4 butanedisulfonate). Interestingly, the two stable salts of SAMe, disulfate-tosylate and 1,4-butanedisulfonate, had identical permeability profiles within experimental error providing the evidence of their biological equivalence. The remaining permeability studies were carried out with SAMe disulfate-tosylate.

The measured permeability values of the SAMe salts were much lower than those measured with the high permeability marker, propranolol (22.4x10$^{-}$6 and 18.4x10$^{-}$6 cms$^{-}$1, respectively.) Permeability coefficients that are concentration independent and/or similar in apical to basolateral as well as basolateral to apical directions are said to be characteristic for paracellular transport. Paracellular transport mechanism for SAMe was supported here by a 13-24 fold increase in SAMe permeability in the calcium-free buffer as well as a 1.3-5.0 fold increase when in the presence of a known tight junction opener, EDTA (as shown in Table 2 and Figure 2).

**Table 2: Permeability of SAMe Across a Monolayer of Caco-2 Cells**

| Well Contents | Apical to Basal $\times 10^{-6}$ cm $s^{-1}$ | | Basal to Apical $\times 10^{-6}$ cm $s^{-1}$ | |
|---|---|---|---|---|
| | Papp_a-b | Papp_a-b SD | Papp_b-a | Papp_b-a SD |
| SAMe 1,4 butanedisulfonate | 0.50 | 0.08 | 0.60 | 0.13 |
| SAMe disulfate tosylate | 0.41 | 0.04 | 0.50 | 0.03 |
| SAMe disulfate tosylate Calcium-free | 9.60 | 0.56 | 6.48 | 0.75 |
| SAMe disulfate tosylate with EDTA | 0.54 | 0.04 | 2.63 | 0.16 |

(continued)

| Well Contents | Apical to Basal x$10^{-6}$ cm $s^{-1}$ | | Basal to Apical x$10^{-6}$ cm $s^{-1}$ | |
|---|---|---|---|---|
| | Papp_a-b | Papp_a-b SD | Papp_b-a | Papp_b-a SD |
| Propranolol (Control) | 22.44 | 1.73 | 18.36 | 0.34 |

Example 3a

SAMe Permeability is increased in the Presence of Tight Junction Modulators

Additional Caco-2 testing was performed on a number of tight junction opening agents at a contract research organization. Similar to the description above, the Caco-2 cell line was obtained from ATCC and grown in DMEM (Sigma-Aldrich) supplemented with 20% FBS (Sigma-Aldrich), 100 uM non-essential amino acids (Sigma-Aldrich) and 2 mM L-glutamine (Sigma-Aldrich). The Caco-2 cells grown in tissue culture flasks were trypsinized, suspended in medium, and the suspensions were applied to wells of a collagen-coated BioCoat Cell Environment in 24-well format at 24,500 cells per well. The cells were allowed to grow and differentiate for three weeks, feeding at 2-day intervals.

For apical to basolateral permeability, a 2 mM aqueous solution of SAMe tosylate disulfate was added to the apical side and the amount of permeation was determined on the basolateral side. The apical and basolateral side buffers contained modified Transport Buffer (25 mM HEPES, 1x Hank's Balanced Salt Solution (Sigm-Aldrich)) pH 7.4. Caco-2 cells were incubated with these buffers for 2 hours, and the receiver side buffer was removed for analysis by LC/MS/MS. The receiver, donor, and dosing solution were diluted with an equal volume of 0.2 N HCl immediately after the assay in order to increase SAMe stability. Donor and dosing solution were diluted 100-fold to ensure that the concentration was within the linear range of the assay.

To confirm the integrity of the Caco-2 cell monolayers, TEER (Trans Epithelial Electrical Resistance) measurements were performed on each well at the end of the experiment.
The permeability (Papp) of SAMe is calculated using the following formula:

$$Papp = \frac{\frac{dQ}{dt}}{CoA}$$

Where dQ/dt is the rate of permeation, $C_0$ is the initial concentration of test agent, and A is the area of the monolayer.

As shown in Table 3 below, the presence of two different fatty acids, either a C10 fatty acid or a sulfonic acid, resulted in a dramatic increase in the permeability of SAMe.

The zwitterionic surfactants, 3-(N,N-Dimethylpalmitylammonio)propanesulfonate and palmitoyl carnitine chloride were also each tested for their effect on SAMe uptake and both showed marked increase in SAMe permeability as seen in Table 3. In addition, alpha-cyclodextrin and a dicarboxcylic acid each resulted in a 5-6 fold increase in SAMe permeability. The low and high permeability controls, ranitidine and warfarin, respectively, verify the use of these absorption enhancing agents as an *in vitro* screening method for measuring the effect of these agents on SAMe permeability across Caco-2 monolayers. Furthermore, the permeability of SAMe alone, versus SAMe in the absence of calcium or in the presence of the tight junction modulators, palmitoyl carnitine chloride or caprate is depicted in the graph in Figure 3.

**Table 3: Permeability of SAMe in the Presence of Various Tight Junction Modulators**

| Test Article | Chemical Class | Caco-2 Concentration | Permeability Coefficient (x$10^{-6}$ cm/s) | Mean TEER Resistance (ohms) | Fold Transport Increase |
|---|---|---|---|---|---|
| SAMe (control) | API control | 2mM | 0.14 (run 2) | 782 | 1X |
| | | | 1.6 (run 3) | 901 | 1X |
| | | | 1.6 (run 4) | 1004 | 1X |
| Sodium Caprate | Fatty acid | 14 mM | 1.79 (run 2) | 235 | 12.8X |
| | | | 9.1 (run 4) | 260 | 5.7X |
| | | 7mM | 4.1 (run 4) | 905 | 2.6X |

(continued)

| Test Article | Chemical Class | Caco-2 Concentration | Permeability Coefficient (x$10^{-6}$ cm/s) | Mean TEER Resistance (ohms) | Fold Transport Increase |
|---|---|---|---|---|---|
| Sodium 1-decanesulfonate | Fatty acid | 14 mM | 3.1 (run 2)<br>15.5 (run 3) | 120<br>135 | 21.9X<br>9.7X |
| | | 7 mM | 6.3 (run 3) | 165 | 3.9X |
| Palmityldimethyl ammonio propane sulfonate | Zwitterionic surfactant | 0.6 mM | 3.8 (run 4) | 415 | 2.4X |
| | | 0.3 mM | 6.0 (run 3)<br>1.4 (run 4) | 420<br>434 | 3.8X<br>0.9X |
| Palmitoyl Carnitine Chloride | Zwitterionic surfactant | 0.15 mM | 0.78 (run 2) | 272 | 5.6X |
| | | | 5.0 (run 4) | 519 | 3.1X |
| | | 0.4 mM | 10.0 (run 4) | 366 | 6.3X |
| Dodecyltrimethyl Ammonium Bromide | Fatty amine | 14 mM | 10.5 (run 4) | 431 | 6.6X |
| Alpha-Cyclodextrin | Cyclodextrin | 51.4 mM | 0.75 (run 2) | 293 | 5.4X |
| | | | 6.3 (run 4) | 341 | 3.9X |
| Dicarboxylic acid | Organic acid | 66.6 mM | 10.5 (run 3) | 143 | 6.6X |
| | | | 15.5 (run 4) | 136 | 9.7X |
| Ranitidine | Low permeability control | 50 uM | 0.91 (run 2) | 1080 | n/a |
| | | | 0.91 (run 3) | 975 | n/a |
| | | | 1.0 (run 4) | 931 | n/a |
| Warfarin | High permeability control | 50 uM | 48.8 (run 2) | 976 | n/a |
| | | | 49.0 (run 3) | 941 | n/a |
| | | | 46.6 (run 4) | 1011 | n/a |

Reference Example 4

Plasma Levels of SAMe Metabolites are not elevated relative to Plasma Levels of SAMe

If SAMe is actively metabolized by the liver upon administration, it would be reasonable to believe that the plasma level of one or more SAMe metabolites would be significantly elevated after administration. In order to test this theory, the present investigators measured the level of S-adenosyl homocysteine (SAH), the primary metabolite of SAMe, at various time points after administration of a 1600 mg dose of a commercially available SAMe formulation.

As seen in Figure 4, at all time points measured, the plasma concentration of SAH (which is plotted on a scale that is tenfold lower than the SAMe concentration) is significantly lower than the level of SAMe itself. Additional SAMe metabolites were also measured and, similarly to SAH, there were no differences in their baseline levels (results not shown.)

These results demonstrate that SAMe metabolism is not responsible for the low bioavailability of SAMe upon administration.

Reference Example 5

SAMe Delivered to the Stomach can be effectively absorbed into the Plasma

Those skilled in these arts are of the view that SAMe delivery must bypass the stomach in order to avoid the harsh low-pH environment which is taught to cause degradation. In order to better understand the mechanism of SAMe absorption, and in order to better control its bioavailability, the release of SAMe into the stomach environment and its

absorption there from was assessed.

Uncoated SAMe was formulated into tablets comprising microcrystalline cellulose, croscarmellose, colloidal silicon dioxide and magnesium stearate using standard procedures as described in Example 1.

The absence of an enteric coating in this formulation was intended to cause release of SAMe within the stomach. The resulting pharmacokinetic profile was studied by measuring the presence of the drug in plasma at various time points after administration. A single dose of 400 mg SAMe was given to seven healthy and fasted, male volunteers.

As seen in Figure 5, the average $C_{max}$ of the seven subjects administered the uncoated SAMe formulation was about 145 ng/mL for the 400 mg dose. These results show that contrary to what is repeatedly reported in the art, SAMe can be delivered to the stomach without using enteric coated formulations and still give rise to significant absorption as seen with the plasma SAMe levels reported here.

## Claims

1. An oral dosage composition comprising a physiologically effective dosage of S-adenosylmethionine in combination with at least one of a zwitterionic surfactant or a salt thereof, a fatty amine or a salt thereof, an alkane sulfonate or a salt thereof, a bile acid or a salt thereof, an unsaturated cyclic urea or a salt thereof, a cyclodextrin or a salt thereof, nonylphenoxypolyoxyethylene, polyoxyethylene sorbitan monolaurate, polyoxyethylene sorbitan monopalmitate, or sorbitan monooleate.

2. The composition of claim 1 wherein the composition comprises S-adenosylmethionine in combination with a zwitterionic surfactant.

3. The composition of claim 1 wherein the composition comprises S-adenosylmethionine in combination with a bile acid.

4. The composition of claim 1 wherein the composition comprises S-adenosylmethionine in combination with an alkane sulfonate.

5. The composition of claim 1 wherein the composition is incorporated in a dietary supplement or a medical food.

6. The composition of any preceding claim, wherein at least a portion of the composition is configured to dissolve in at least one of the stomach, duodenum, jejunum and ileum.

7. The composition of any preceding claim wherein at least a portion of the composition is configured to dissolve in the large intestine or colon.

8. The composition of claim 6 or 7 wherein the composition incorporates a pH sensitive coating.

9. The composition of any preceding claim for use in treating in a patient a disorder selected from the group consisting of a mental or psychiatric disorder, a nervous system disease/disorder, other neurological disease/disorders, conditions associated with injury to the central nervous system, a liver disease/disorder, a cancer, a joint disease/disorder, an inflammatory disease/disorder, an autoimmune disease/disorder, a degenerative disease/disorder, a soft-tissue disease/disorder, a pain disease/disorder, a genetic disorder related to hyper- or hypo-methylation, a gastrointestinal disease/disorder, a cardiovascular disease/disorder, and a disorder induced in whole or in part by oxidative or free-radical damage, the composition comprising a physiologically effective dosage of S-adenosylmethionine in combination with at least one of a zwitterionic surfactant or a salt thereof, a fatty amine or a salt thereof, an alkane sulfonate or a salt thereof, a bile acid or a salt thereof, an unsaturated cyclic urea or a salt thereof, a cyclodextrin or a salt thereof, nonylphenoxypolyoxyethylene, polyoxyethylene sorbitan monolaurate, polyoxyethylene sorbitan monopalmitate, or sorbitan monooleate.

10. The oral composition for use in claim 9, wherein

    (i) the mental or psychiatric disorder is selected from the group consisting of an anxiety disorder, schizophrenia, major depressive disorder, multi-infarct dementia, minor depression, postpartum depression, late-life depression, Parkinson's depression, HIV-associated depression, and bipolar disorder;
    (ii) the inflammatory disease or disorder is selected from the group consisting of systemic lupus, inflammatory bowel disease, allergic rhinitis, contact dermatitis, asthma, autoimmune hepatitis, and pelvic inflammatory disease;

(iii) the cardiovascular disease or disorder is selected from the group consisting of hyper- or hypo-homocysteinemia, coronary heart disease, stroke, peripheral vascular disease, and atherosclerotic disease;
(iv) the depressive disorder is a comorbid depression arising in a subject who is or has been undergoing treatment for one or more diseases or disorders selected from the group consisting of cancer, Parkinson's and HIV;
(v) the nervous system disease or disorder or injury is selected from the group consisting of Parkinson's disease, Alzheimer's disease, and cognitive impairment;
(vi) the liver disease or disorder is selected from the group consisting of alcoholic liver disease, non-alcoholic fatty liver disease, viral or non-viral hepatitis, liver cancer, oxidative liver disease, cholestasis, and cirrhosis;
(vii) the cancer is selected from the group consisting of liver cancer, colon cancer, rectal cancer, stomach cancer, esophageal cancer, and adenocarcinoma;
(viii) the joint disease or disorder is arthritis or osteoarthritis;
(ix) the soft-tissue disease or disorder is fibromyalgia;
(x) the pain disease or disorder is fibromyalgia; or
(xi) the genetic disorder related to hyper- or hypo-methylation is methylenetetrahydrofolate reductase deficiency.

**11.** The composition for use in claim 9 or 10, further comprising an active ingredient that is used for treatment or prophylaxis of a mental or psychiatric disorder.

**12.** The composition for use in claim 11, wherein said active ingredient is selected from the group consisting of a tricyclic antidepressant (TCA), a tetracyclic antidepressant, an aminoketone, a phenylpiperazine, a selective serotonin reuptake inhibitor (SSRI), a monoamine oxidase inhibitors (MAOI), a serotonin-norepinephrine reuptake inhibitor (SNRI), a norepinephrine-serotonin reuptake inhibitor (NSRI), a dopamine reuptake inhibitor, a norepinephrine-dopamine reuptake inhibitor, a norepinephrine reuptake inhibitor, a selective serotonin reuptake enhancer, a noradrenergic and serotonin specific antidepressant, a substance P receptor antagonist, a neurokinin receptor antagonist, saredutant, a corticotrophin release factor antagonist, mifepristone, an atypical antipsychotic, aripiparazole, lithium, and a triple reuptake inhibitor.

## Patentansprüche

**1.** Orale Dosierungszusammensetzung umfassend eine physiologisch wirksame Dosierung von S-Adenosylmethionin in Kombination mit zumindest einem aus einem zwitterionischen Tensid oder einem Salz davon, einem Fettamin oder einem Salz davon, einem Alkansulfonat oder einem Salz davon, einer Gallensäure oder einem Salz davon, einem ungesättigten zyklischen Harnstoff oder einem Salz davon, einem Zyklodextrin oder einem Salz davon, Nonylphenoxypolyoxyethylen, Polyoxyethylen-Sorbitanmonolaurat, Polyoxyethylen-Sorbitanmonopalmitat oder Sorbitanmonooleat.

**2.** Zusammensetzung nach Anspruch 1, worin die Zusammensetzung S-Adenosylmethionin in Kombination mit einem zwitterionischen Tensid umfasst.

**3.** Zusammensetzung nach Anspruch 1, worin die Zusammensetzung S-Adenosylmethionin in Kombination mit einer Gallensäure umfasst.

**4.** Zusammensetzung nach Anspruch 1, worin die Zusammensetzung S-Adenosylmethionin in Kombination mit einem Alkansulfonat umfasst.

**5.** Zusammensetzung nach Anspruch 1, worin die Zusammensetzung in ein Nahrungsergänzungsmittel oder ein medizinisches Nahrungsmittel aufgenommen ist.

**6.** Zusammensetzung nach irgendeinem vorhergehenden Anspruch, worin zumindest ein Teil der Zusammensetzung dazu konfiguriert ist, sich in zumindest einem des Magens, Duodenums, Jejunums und Ileums aufzulösen.

**7.** Zusammensetzung nach irgendeinem vorhergehenden Anspruch, worin zumindest ein Teil der Zusammensetzung dazu konfiguriert ist, sich im Dickdarm oder Kolon aufzulösen.

**8.** Zusammensetzung nach Anspruch 6 oder 7, worin die Zusammensetzung eine pHempfindliche Beschichtung enthält.

**9.** Zusammensetzung nach irgendeinem vorhergehenden Anspruch zur Verwendung bei der Behandlung eines Patienten gegen eine Krankheit, die aus der Gruppe ausgewählt wird, die aus einer geistigen oder psychiatrischen Störung, einer Nervensystemkrankheit/-störung, sonstigen neurologischen Krankheiten/Störungen, mit Verletzung des zentralen Nervensystems assoziierten Leiden, einer Leberkrankheit/-störung, einem Krebs, einer Gelenkkrankheit/-störung, einer entzündlichen Krankheit/Störung, einer Autoimmunkrankheit/-störung, einer degenerativen Krankheit/Störung, einer Weichgewebe-Krankheit/Störung, einer Schmerzkrankheit/-störung, einer mit Hyper- oder Hypomethylation verwandten genetischen Störung, einer gastrointestinalen Krankheit/Störung, einer kardiovaskulären Krankheit/Störung und einer Störung, die ganz oder teilweise durch oxidative oder Freie Radikale-Schädigung induziert wird, besteht, wobei die Zusammensetzung eine physiologisch wirksame Dosierung von S-Adenosylmethionin in Kombination mit zumindest einem aus einem zwitterionischen Tensid oder einem Salz davon, einem Fettamin oder einem Salz davon, einem Alkansulfonat oder einem Salz davon, einer Gallensäure oder einem Salz davon, einem ungesättigten zyklischen Harnstoff oder einem Salz davon, einem Zyklodextrin oder einem Salz davon, Nonylphenoxypolyoxyethylen, Polyoxyethylen-Sorbitanmonolaurat, Polyoxyethylen-Sorbitanmonopalmitat oder Sorbitanmonooleat umfasst.

**10.** Orale Zusammensetzung zur Verwendung in Anspruch 9, worin

(i) die geistige oder psychiatrische Störung aus der Gruppe ausgewählt wird, die aus einer Angststörung, Schizophrenie, schweren depressiven Störung, Multünfarkt-Demenz, leichten Depression, postpartalen Depression, Altersdepression, Parkinson-Depression, HIV-assoziierten Depression und bipolaren Störung besteht;
(ii) die entzündliche Krankheit oder Störung aus der Gruppe ausgewählt wird, die aus systemischem Lupus, entzündlicher Darmkrankheit, allergischer Rhinitis, Kontaktdermatitis, Asthma, autoimmuner Hepatitis und entzündlicher Beckenkrankheit besteht;
(iii) die kardiovaskuläre entzündliche Krankheit oder Störung aus der Gruppe ausgewählt wird, die aus Hyper- oder Hypo-Homocysteinämie, koronarer Herzkrankheit, Schlaganfall, peripherer vaskulärer Krankheit und atherosklerotischer Krankheit besteht;
(iv) die depressive Störung eine comorbide Depression ist, die bei einem Patienten auftritt, der gegen eine oder mehrere, aus der aus Krebs, Parkinson und HIV bestehenden Gruppe ausgewählte Krankheiten oder Störungen behandelt wird oder worden ist;
(v) die Nervensystemkrankheit oder -Störung oder -Verletzung aus der Gruppe ausgewählt wird, die aus Parkinson-Krankheit, Alzheimer-Krankheit und kognitiver Störung besteht;
(vi) die Leberkrankheit oder -Störung aus der Gruppe ausgewählt wird, die aus alkoholischer Leberkrankheit, nicht-alkoholischer Fettleberkrankheit, viraler oder non-viraler Hepatitis, Leberkrebs, oxidativer Leberkrankheit, Cholestase und Zirrhose besteht;
(vii) der Krebs aus der Gruppe ausgewählt wird, die aus Leberkrebs, Kolonkrebs, Rektumkrebs, Magenkrebs, Speiseröhrenkrebs und Adenokarzinom besteht;
(viii) die Gelenkkrankheit oder -Störung Arthritis oder Osteoarthritis ist;
(ix) die Weichgewebekrankheit oder -Störung Fibromyalgie ist;
(x) die Schmerzkrankheit oder -Störung Fibromyalgie ist; oder
(xi) die mit Hyper- oder Hypomethylation verwandte genetische Störung ein Methylentetrahydrofolat-Reduktase-Mangel ist.

**11.** Zusammensetzung zur Verwendung in Anspruch 9 oder 10, ferner umfassend einen Wirkstoff, der zur Behandlung oder Prophylaxe einer geistigen oder psychiatrischen Störung verwendet wird.

**12.** Zusammensetzung zur Verwendung in Anspruch 11, worin besagter Wirkstoff aus der Gruppe ausgewählt wird, die aus einem trizyklischen Antidepressivum (TCA), einem tetrazyklischen Antidepressivum, einem Aminoketon, einem Phenylpiperazin, einem selektiven Serotonin-Wiederaufnahmehemmer (SSRI), einem Monoaminoxidase-Hemmer (MAOI), einem Serotonin-Norepinephrin-Wiederaufnahmehemmer (SNRI), einem Norepinephrin-Serotonin-Wiederaufnahmehemmer (NSRI), einem Dopamin-Wiederaufnahmehemmer, einem Norepinephrin-Dopamin-Wiederaufnahmehemmer, einem Norepinephrin-Wiederaufnahmehemmer, einem selektiven Serotonin-Wiederaufnahmeverstärker, einem noradrenergenen und serotoninspezifischen Antidepressivum, einem Substanz-P-Rezeptorantagonisten, einem Neurokinin-Rezeptorantagonisten, Saredutant, einem Corticotrophin-Freisetzungsfaktor-Agonisten, Mifepriston, einem atypischen Antipsychotikum, Aripiparazol, Lithium und einem dreifachen Wiederaufnahmehemmer besteht.

## Revendications

1. Une composition administrée par voie orale comprenant une dose physiologiquement efficace de S-adénosylméthionine en combinaison avec au moins soit un agent tensioactif zwittérionique ou un de ses sels, soit une amine grasse ou un de ses sels, soit un sulfonate d'alcane ou un de ses sels, soit un acide biliaire ou un de ses sels, soit une urée cyclique insaturée ou un de ses sels, soit une cyclodextrine ou un de ses sels, soit du nonylphénoxypoly(oxyéthylène), soit du monolaurate de sorbitan polyoxyéthyléné, soit du monopalmitate de sorbitan polyoxyéthyléné, soit du monooléate de sorbitan.

2. La composition selon la revendication 1 dans laquelle la composition comprend de la S-adénosylméthionine en combinaison avec un agent tensioactif zwittérionique.

3. La composition selon la revendication 1 dans laquelle la composition comprend de la S-adénosylméthionine en combinaison avec un acide biliaire.

4. La composition selon la revendication 1 dans laquelle la composition comprend de la S-adénosylméthionine en combinaison avec un sulfonate d'alcane.

5. La composition selon la revendication 1 dans laquelle la composition est incorporée dans un complément alimentaire ou un aliment médical.

6. La composition selon l'une quelconque des revendications précédentes, dans laquelle au moins une partie de la composition est configurée pour se dissoudre dans au moins soit l'estomac, soit le duodénum, soit le jéjunum, soit l'iléum.

7. La composition selon l'une quelconque des revendications précédentes dans laquelle au moins une partie de la composition est configurée pour se dissoudre dans le gros intestin ou le colon.

8. La composition selon la revendication 6 ou 7 dans laquelle la composition incorpore un revêtement sensible au pH.

9. La composition selon l'une quelconque des revendications précédentes pour une utilisation dans le traitement chez un patient d'un trouble sélectionné dans le groupe constitué par un trouble mental ou psychiatrique, une maladie/un trouble du système nerveux, d'autres maladies/troubles neurologiques, des conditions associées à une lésion du système nerveux central, une maladie/un trouble du foie, un cancer, une maladie/un trouble des articulations, une maladie/un trouble inflammatoire, une maladie/un trouble auto-immune, une maladie/un trouble dégénératif, une maladie/un trouble des tissus mous, une maladie/un trouble de la douleur, un trouble génétique relatif à l'hyper- ou l'hypo-méthylation, une maladie/un trouble gastro-intestinal, une maladie/un trouble cardiovasculaire et un trouble induit en totalité ou en partie par les dommages des oxydants ou des radicaux libres, la composition comprenant une dose physiologiquement efficace de S-adénosylméthionine en combinaison avec au moins soit un agent tensioactif zwittérionique ou un de ses sels, soit une amine grasse ou un de ses sels, soit un sulfonate d'alcane ou un de ses sels, soit un acide biliaire ou un de ses sels, soit une urée cyclique insaturée ou un de ses sels, soit une cyclodextrine ou un de ses sels, soit du nonylphénoxy-poly(oxyéthylène), soit du monolaurate de sorbitan polyoxyéthyléné, soit du monopalmitate de sorbitan polyoxyéthyléné, soit du monooléate de sorbitan.

10. La composition orale utilisée dans la revendication 9, dans laquelle

(i) le trouble mental ou psychiatrique est sélectionné dans le groupe constitué par un trouble de l'anxiété, une schizophrénie, un trouble dépressif majeur, une démence vasculaire, une dépression mineure, une dépression post-partum, une dépression du grand âge, une dépression de Parkinson, une dépression associée au VIH et un trouble bipolaire ;
(ii) la maladie ou le trouble inflammatoire est sélectionné dans le groupe constitué par le lupus systémique, la maladie inflammatoire intestinale, la rhinite allergique, la dermatite de contact, l'asthme, l'hépatite auto-immune et la maladie inflammatoire du pelvis ;
(iii) la maladie ou le trouble cardiovasculaire est sélectionné dans le groupe constitué par l'hyper- ou l'hyperhomocystéinémie, la maladie cardiaque coronaire, l'accident vasculaire cérébral, la maladie vasculaire périphérique et la maladie athérosclérotique ;
(iv) le trouble dépressif est une dépression comorbide se présentant chez un sujet qui subit ou a subi un traitement pour au moins une maladie ou un trouble sélectionné dans le groupe constitué par le cancer, la

maladie de Parkinson et le VIH ;
(v) la maladie ou le trouble ou la lésion du système nerveux est sélectionné dans le groupe constitué par la maladie de Parkinson, la maladie d'Alzheimer et la déficience cognitive ;
(vi) la maladie ou le trouble du foie est sélectionné dans le groupe constitué par la maladie du foie alcoolique, la maladie du foie gras non alcoolique, l'hépatite virale ou non virale, le cancer du foie, la maladie du foie de l'oxydation, la cholestase et la cirrhose ;
(vii) le cancer est sélectionné dans le groupe constitué par le cancer du foie, le cancer du colon, le cancer rectal, le cancer de l'estomac, le cancer de l'oesophage et l'adénocarcinome ;
(viii) la maladie ou le trouble des articulations est l'arthrite ou l'ostéoarthrite ;
(ix) la maladie ou le trouble des tissus mous est une fibromyalgie ;
(x) la maladie ou le trouble de la douleur est une fibromyalgie ; ou
(xi) le trouble génétique relatif à l'hyper- ou l'hypo-méthylation est une carence en méthylène-tétrahydrofolate réductase.

**11.** La composition utilisée dans la revendication 9 ou 10, comprenant en outre un ingrédient actif qui est utilisé pour le traitement ou la prophylaxie d'un trouble mental ou psychiatrique.

**12.** La composition utilisée dans la revendication 11, dans laquelle ledit ingrédient actif est sélectionné dans le groupe constitué par un antidépresseur tricyclique (ATC), un antidépresseur tétracyclique, un aminocétone, un phénylpipérazine, un inhibiteur sélectif de la recapture de la sérotonine (ISRS), des inhibiteurs de la monoamine oxydase (IMAO), un inhibiteur de la recapture de la sérotoninenoradrénaline (IRSN), un inhibiteur de la recapture de la norépinéphrinesérotonine (IRNS), un inhibiteur de la recapture de la dopamine, un inhibiteur de la recapture de la norépinéphrine-dopamine, un inhibiteur de la recapture de la norépinéphrine, un inhibiteur sélectif de la recapture de la sérotonine, un antidépresseur noradrénergique et sérotoninergique spécifique, un antagoniste des récepteurs de la substance P, un antagoniste des récepteurs de la neurokinine, le sarédutant, un antagoniste du facteur de libération de la corticotrophine, le mifépristone, un antipsychotique atypique, l'aripiparazole, le lithium et un inhibiteur de recapture triple.

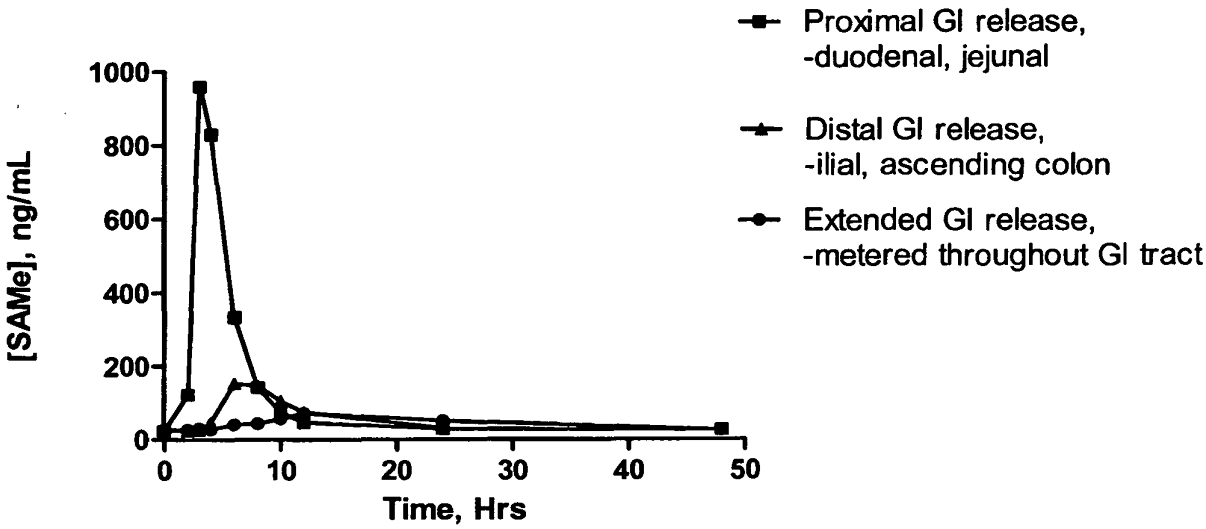
[SAMe], ng/mL
1000
800
600
400
200
0
0
10
20
30
40
50
Time, Hrs
Proximal GI release,
-duodenal, jejunal
Distal GI release,
-ilial, ascending colon
Extended GI release,
-metered throughout GI tract

FIGURE 1A

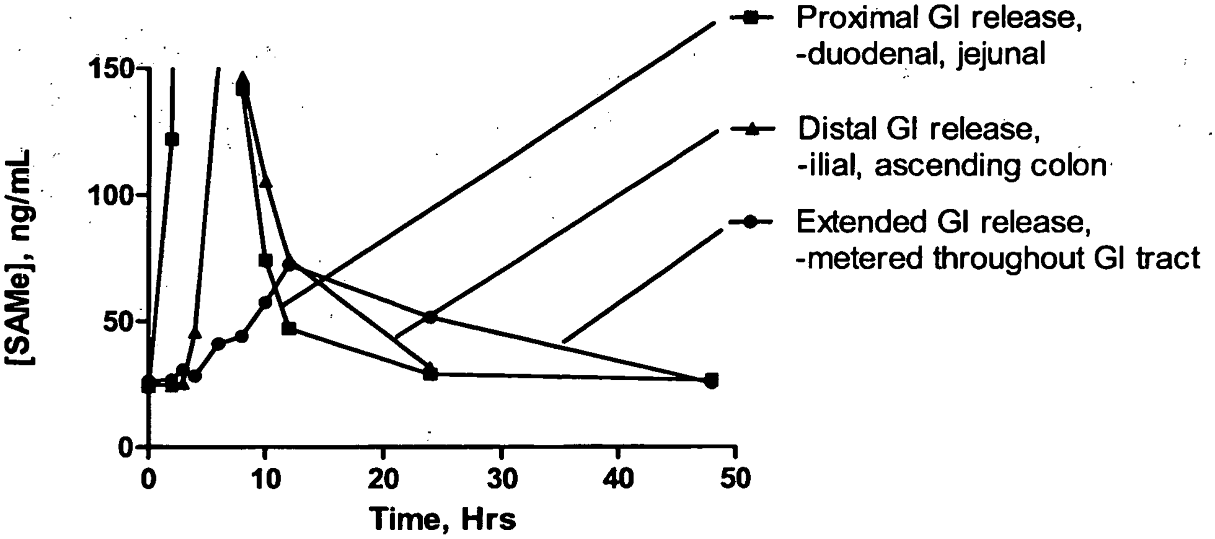
[SAMe], ng/mL
150
100
50
0
0
10
20
30
40
50
Time, Hrs
Proximal GI release,
-duodenal, jejunal
Distal GI release,
-ilial, ascending colon
Extended GI release,
-metered throughout GI tract

FIGURE 1B

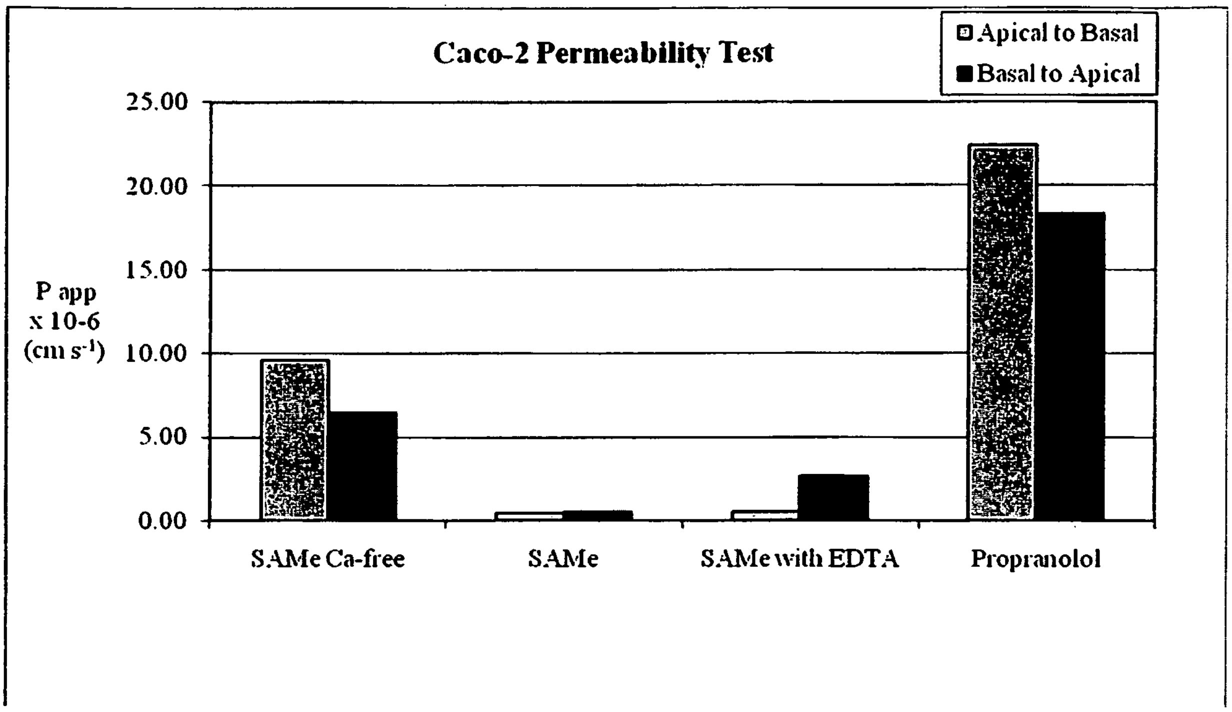
Caco-2 Permeability Test
Apical to Basal
Basal to Apical
25.00
20.00
15.00
10.00
5.00
0.00
P app
x 10-6
(cm s-1)
SAMe Ca-free
SAMe
SAMe with EDTA
Propranolol

**FIGURE 2**

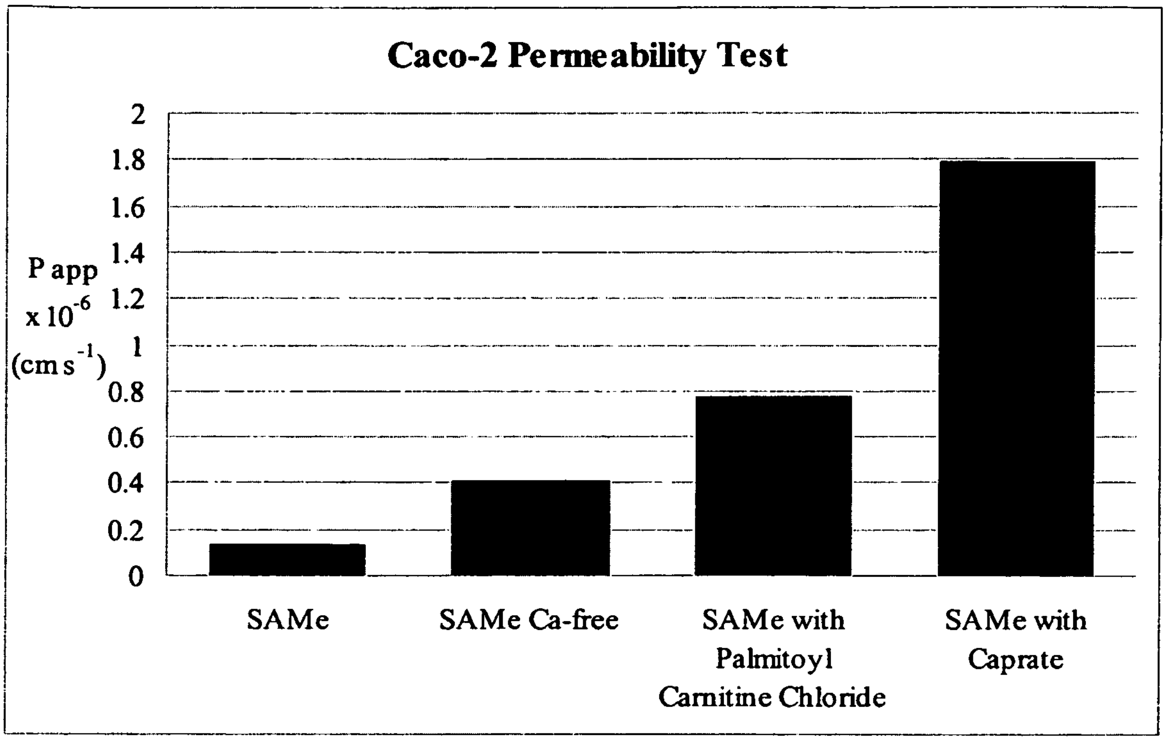
Caco-2 Permeability Test
P app x 10-6 (cm s-1)
2
1.8
1.6
1.4
1.2
1
0.8
0.6
0.4
0.2
0
SAMe
SAMe Ca-free
SAMe with Palmitoyl Carnitine Chloride
SAMe with Caprate

**FIGURE 3**

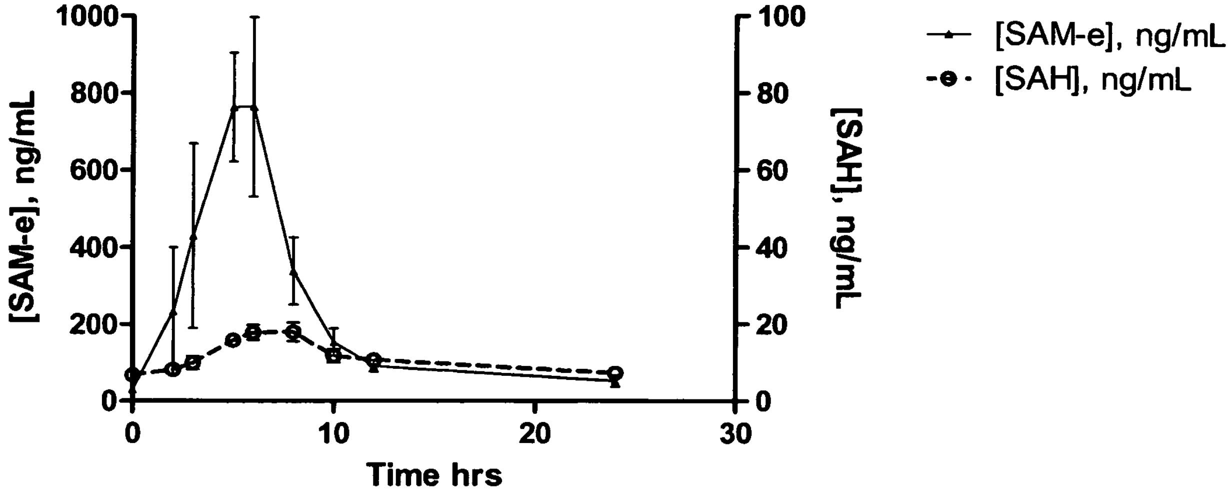
1000
800
600
400
200
0
[SAM-e], ng/mL
100
80
60
40
20
0
[SAH], ng/mL
0
10
20
30
Time hrs
[SAM-e], ng/mL
[SAH], ng/mL

FIGURE 4

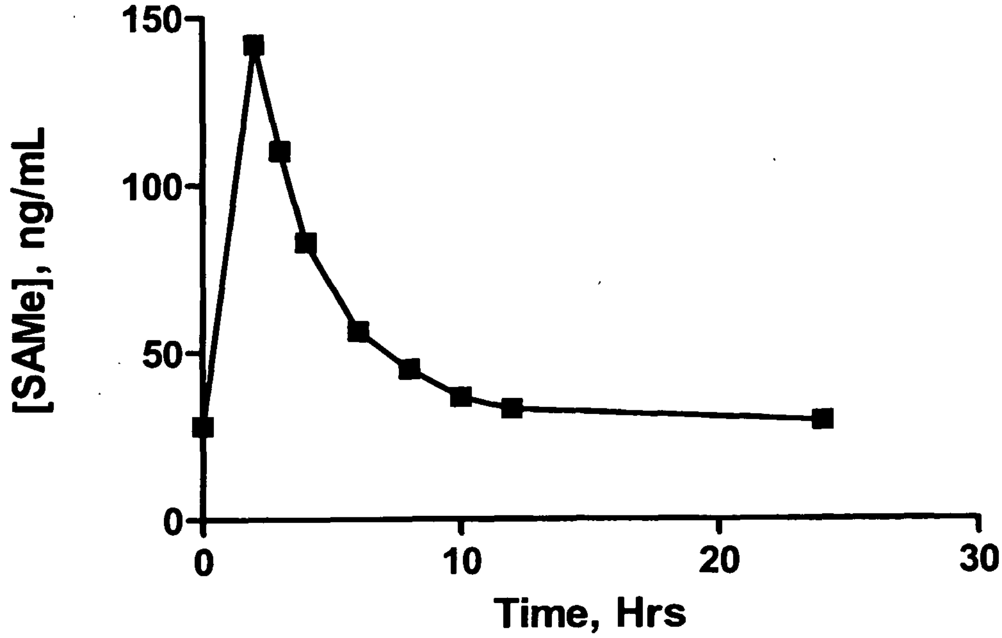


FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3954726 A
- US 4057686 A
- US 6759395 B
- US 2002164369 A
- US 2002013905 A
- US 4956173 A
- US 3893999 A
- US 5128249 A
- US 20090088404 A


### Non-patent literature cited in the description


- **STRAMENTINOLI, G.** *The American Journal of Medicine,* 1987, vol. 83 (S 5A), 35-42
- **BOTTIGLIERI et al.** *Alabama Journal of Medical Sciences,* 1988, vol. 25 (3), 296-301
- **BOTTIGLIERI et al.** *Exp. Opin. Invest. Drugs,* 1997, vol. 6 (4), 417-426
- **KAYE et al.** *Drugs,* 1990, vol. 40, 124-128
- **GIULIDORI ; CORTELLARO.** *European Journal of Clinical Pharmacology,* 1984, vol. 27, 119-121
- **STRAMENTINOLI, G.** Biological Methylation and Drug Design. Humana Press, 1986, 315-326
- **MCMILLAN et al.** *J. of Pharmacy and Pharmacology,* 2005, vol. 57, 599